# EUROPEAN PATENT SPECIFICATION

(11) **EP 4 001 888 B1**
(45) Date of publication and mention of the grant of the patent: **04.10.2023**
(21) Application number: 20859017.4
(22) Date of filing: 24.08.2020
(51) Int. Cl.: G01N 1/30, G01N 33/48, G01N 33/58

(54) **BIOLOGICAL TISSUE STAINING REAGENT, BIOLOGICAL TISSUE STAINING KIT AND BIOLOGICAL TISSUE STAINING METHOD**
FÄRBEREAGENZ FÜR BIOLOGISCHES GEWEBE, FÄRBEKIT FÜR BIOLOGISCHES GEWEBE UND FÄRBEVERFAHREN FÜR BIOLOGISCHES GEWEBE
RÉACTIF DE COLORATION DE TISSU BIOLOGIQUE, NÉCESSAIRE DE COLORATION DE TISSU BIOLOGIQUE ET MÉTHODE DE COLORATION DE TISSU BIOLOGIQUE

(30) Priority: 30.08.2019 JP 2019157984; 18.03.2020 JP 2020047552
(43) Date of publication of application: 25.05.2022
(73) Proprietor: Cubicstars, Inc., Kurume-shi Fukuoka 839-0864 (JP)
(72) Inventor: UEDA Hiroki, Kurume-shi, Fukuoka 839-0864 (JP); SUSAKI Etsuo, Kurume-shi, Fukuoka 839-0864 (JP)
(74) Representative: Graf von Stosch Patentanwaltsgesellschaft mbH
(86) International application number: PCT/JP2020/031840
(87) International publication number: WO 2021/039716

(56) References cited:
- WO-A1-2015/022883
- CN-A- 102 589 955
- JP-A- 2003 066 035
- KR-A- 20110 121 876
- US-A1- 2004 137 475

## Description

### Technical Field

The present invention relates to a biological tissue-staining reagent, a biological tissue-staining kit, and a biological tissue-staining method.

### Background Art

In medical and biological research, systematic three-dimensional (3D) observations and analyses have been attempted, for example, at the whole-tissue level and the whole-individual level. Development of methods of clearing and 3D imaging of tissues has enabled comprehensive observation of the entire body or entire organ at the single-cell resolution or a finer resolution. In order to obtain more biological information from such datasets, labels suitable for structures, cell types, and cell activities are required.

For the labeling, methods utilizing genetics and viruses have been used. Further, a variety of dyes and antibodies in combination with clearing and 3D imaging have been used to evaluate biological specimens by 3D staining. Staining using dyes and antibodies is applicable to a wide range of samples including human and animal specimens, and relatively easily enables labeling of a plurality of targets. Therefore, compared to the methods using genetics and viruses, such staining is more widely applicable and more advantageous. In practice, from the 1980s, systematic observation of entire organs and entire bodies has been studied for insects, shrimp nervous systems, frog embryos, and the like. In recent years, 3D observation has been developed for a variety of organs, whole bodies, pathological specimens, and the like of rodents, humans, and the like.

Several methods are available for improvement of penetration of dyes and antibodies to the inside of thick specimens. For example, permeabilization treatment by expansion of spores in fixed tissues has been attempted using defatting treatment, dehydration treatment, mild fixation treatment, partial degradation with protease, and the like. For controlling binding affinity of the dyes and antibodies during the penetration, urea or SDS is used. Further, physical methods such as electrophoresis and pressurization are applied to samples embedded in acrylamide.

Patent Literature 1 discloses an immunostaining method in which an antibody composition containing: a predetermined concentration of urea; and an immunostaining antibody; is brought into contact with a biological material. Non Patent Literature 1 and 2 disclose, as an immunostaining method, the iDISCO method, in which a sample treated with methanol or dimethyl sulfoxide (DMSO) is incubated in a permeabilization solution containing glycine, DMSO, and the like, and then blocking treatment is carried out, followed by allowing reaction with a primary antibody in a solution containing heparin, DMSO, donkey serum, and the like, and then allowing reaction with a secondary antibody.

### Citation List

### Patent Literature

Patent Literature 1: International Publication No. WO 2014/010633

### Non Patent Literature

Non Patent Literature 1: Nicolas Renier and five other authors, "iDISCO: a simple, rapid method to immunolabel large tissue samples for volume imaging.", Cell, 2014, 159, 896-910
Non Patent Literature 2: Nicolas Renier and 15 other authors, "Mapping of brain activity by automated volume analysis of immediate early genes.", Cell, 2016, 165, 1789-1802

### Summary of Invention

### Technical Problem

Neither the antibody composition disclosed in Patent Literature 1, nor the methods for improving penetration of dyes and antibodies including the iDISCO method disclosed in Non Patent Literature 1 and 2, has achieved a sufficient penetration efficiency. In cases of tissue staining of a thick sample, even a low-molecular-weight dye may hardly penetrate into the tissue because of a complex physicochemical environment of the staining target.

Further, the iDISCO method disclosed in Non Patent Literature 1 and 2 may cause fading of fluorescent proteins such as Green Fluorescent Protein (GFP), or shrinkage of samples during dehydration and clearing treatments. Thus, the iDISCO method has a disadvantageous aspect in the use for analysis of a biological tissue in which a fluorescent protein is expressed.

The present invention was made in view of the above circumstances, and an objective of the disclosure is to provide a biological tissue-staining reagent, a biological tissue-staining kit, and a biological tissue-staining method that are applicable to a wide range of biological tissues, and that allow sufficient penetration of dyes and antibodies.

### Solution to Problem

The present inventors discovered that a fixed and defatted biological tissue can be characterized as an anionically charged electrolyte gel mainly including cross-linked polypeptide, and that the chemical properties of the biological tissue can be defined as an electrolyte gel. Focusing on the penetration of a dye or an immunostaining antibody into the electrolyte gel in relation to the salt concentration and the composition of the buffer containing the dye or the antibody, and in relation to the experimental conditions; the present inventors repeated trial and error, to complete the present disclosure.

A biological tissue-staining reagent according to a first aspect of the present invention includes:
a nonionic surfactant at a concentration higher than 1%; and
a salt at not less than 200 mM.

In this case, the biological tissue-staining reagent according to the first aspect of the present invention may further include a neutral buffer.

The biological tissue-staining reagent according to the first aspect of the present disclosure may further include a blocking reagent.

The biological tissue-staining reagent according to the first aspect of the present invention may further include at least one additive selected from compounds of aromatic amines, aliphatic amides, nicotinamides, sulfamides, sulfonic acid salts, amino alcohols, alcohols, sulfinic acids, thioureas, and carboxylic acids except urea and urea derivatives, wherein the compounds are represented by the following General Formula (1): 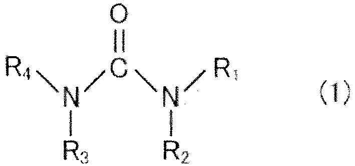 (where in General Formula (1), R₁, R₂, R₃, and R₄ are each independently a hydrogen atom, a halogen atom, or a hydrocarbon group, wherein, in a case where a plurality of carbon atoms constitutes the hydrocarbon group, part of the carbon atoms are optionally substituted by a heteroatom such as a nitrogen atom, an oxygen atom, or a sulfur atom, and wherein the hydrocarbon group includes chain hydrocarbon groups and cyclic hydrocarbon groups).

The biological tissue-staining reagent may further include a dye.

The biological tissue-staining reagent may further include an immunostaining antibody.

A biological tissue-staining kit according to a second aspect of the present invention includes:
a biological tissue-staining reagent according to the first aspect of the present disclosure; and
a dye.

A biological tissue-staining kit according to a third aspect of the present invention includes:
a biological tissue-staining reagent according to the first aspect of the present disclosure; and
an immunostaining antibody.

In this case, the biological tissue-staining kit may further include a slightly acidic buffer.

The biological tissue-staining kit may further include a washing buffer containing a nonionic surfactant at a concentration higher than 1%, a salt at not less than 200 mM, and a neutral buffer.

The biological tissue-staining kits may further include a phase separation-inducing reagent for phase separation from water.

A biological tissue-staining method according to a fourth aspect of the present invention is a biological tissue-staining method using the biological tissue-staining reagent according to the first aspect of the present disclosure, the method including:
exposing a defatted biological tissue to a slightly acidic buffer containing the immunostaining antibody; and
exposing the biological tissue to the biological tissue-staining reagent.

A biological tissue-staining method according to a fifth aspect of the present invention includes:
exposing a biological tissue to a biological tissue-staining reagent according to the first aspect of the present invention, the reagent including a dye and an immunostaining antibody.

A biological tissue-staining method according to a sixth aspect of the present invention includes:
mixing the biological tissue-staining reagent according to the first aspect of the present disclosure, the reagent including at least one of a dye or an immunostaining antibody, a biological tissue, and a phase separation-inducing reagent.

### Advantageous Effects of Invention

The present disclosure is applicable to a wide range of biological tissues, allows sufficient penetration of dyes and antibodies.

### Brief Description of Drawings

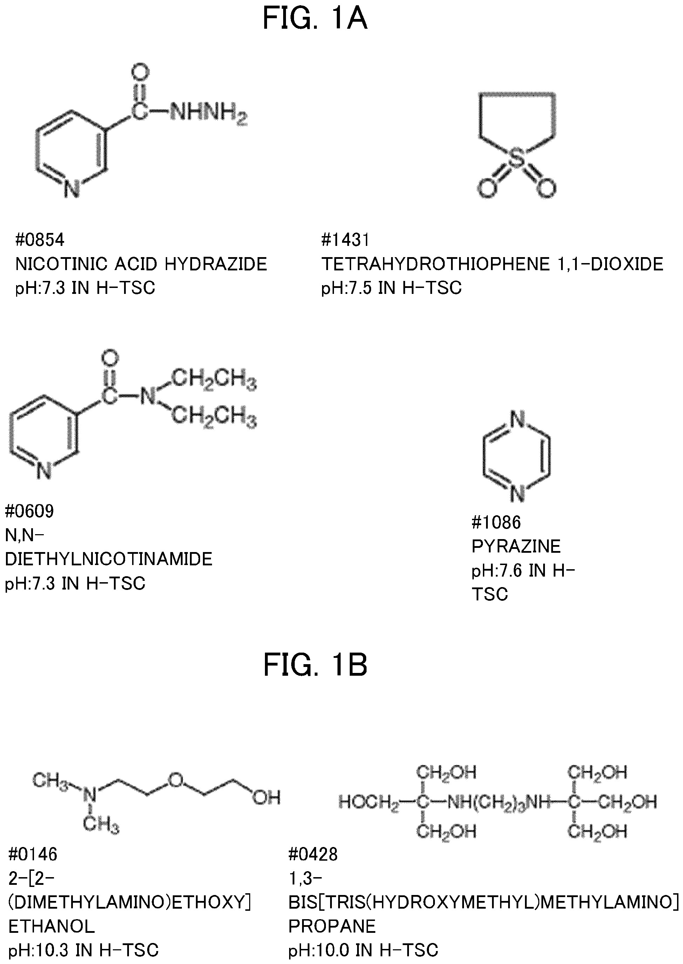

FIG. 1 is a diagram showing representative additives in embodiments according to the present disclosure, wherein FIG. 1A is a diagram showing additives having neutral pHs; and FIG. 1B is a diagram showing additives having alkaline pHs;

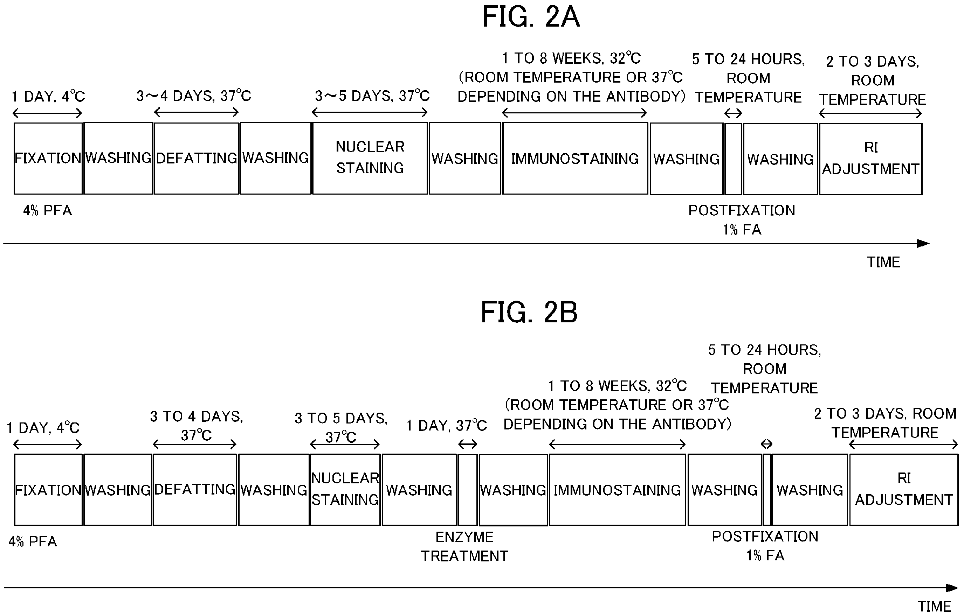

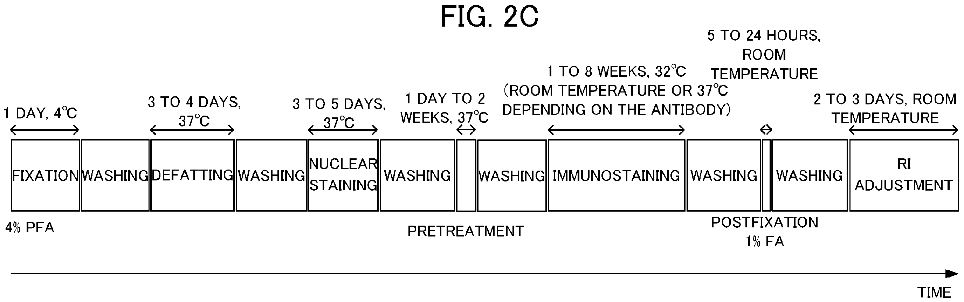

FIG. 2 is a diagram showing the steps of biological tissue-staining methods of embodiments according to the present disclosure, wherein the steps are presented along the time axis, wherein FIG. 2A is a diagram showing an example of a biological tissue-staining method according to an embodiment; FIG. 2B is a diagram showing a biological tissue-staining method that is the same as the biological tissue-staining method shown in FIG. 1A except that an enzyme treatment step is added; and FIG. 2C is a diagram showing a biological tissue-staining method that is the same as the biological tissue-staining method shown in FIG. 1A except that a pretreatment step is added;

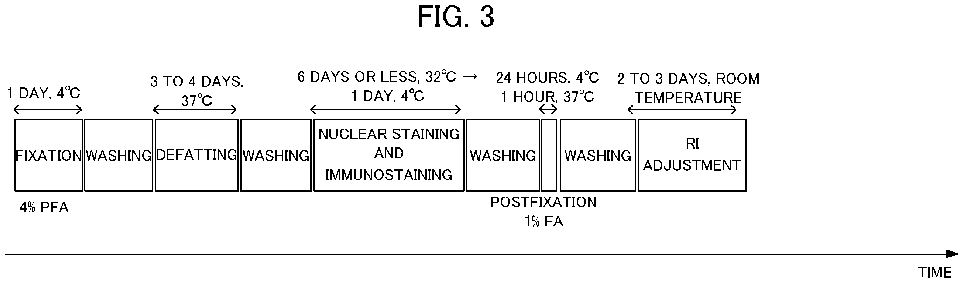

FIG. 3 is a diagram showing the steps of a biological tissue-staining method of another embodiment according to the present disclosure, wherein the steps are presented along the time axis;

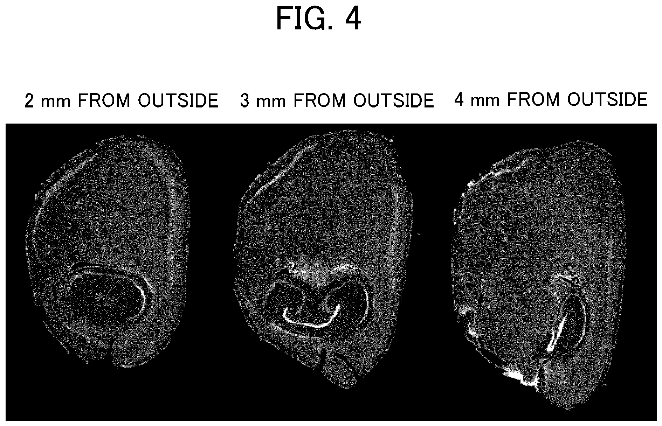

FIG. 4 is a diagram showing images of frozen sections prepared from mouse cerebral hemisphere subjected to 3D staining with a dye in Example 1;

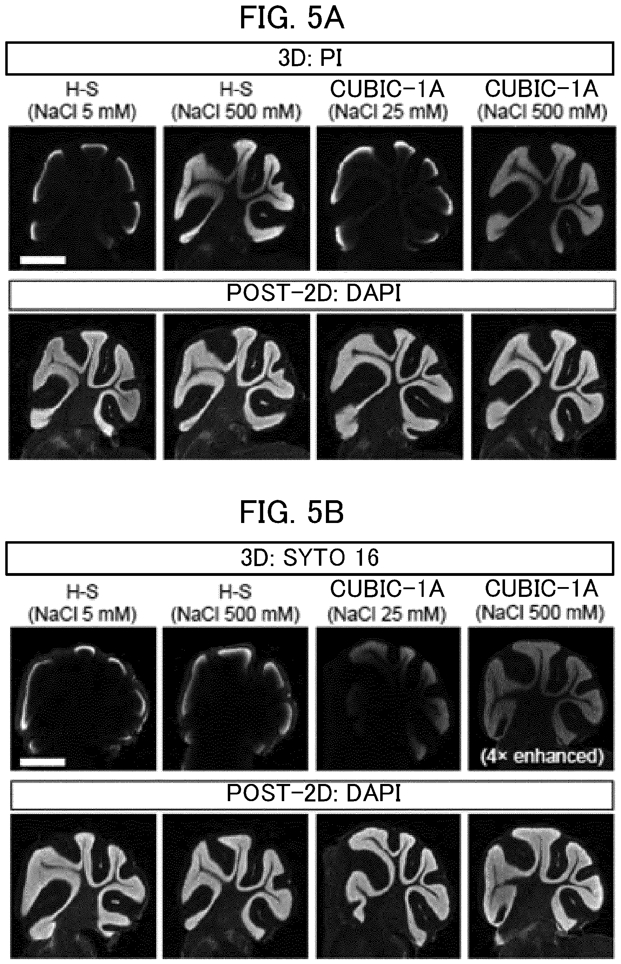

FIG. 5 is a diagram showing an image of a frozen section prepared from mouse cerebellar hemisphere subjected to 3D staining with a dye in Example 2, wherein FIG. 5A and FIG. 5B are diagrams showing images of frozen sections prepared from mouse cerebellar hemisphere subjected to 3D staining with propidium iodide (hereinafter referred to as "PI") or SYTO (trademark) 16 (hereinafter referred to as "SYTO 16"); and each scale bar in FIG. 5A and FIG. 5B corresponds to 1 mm;

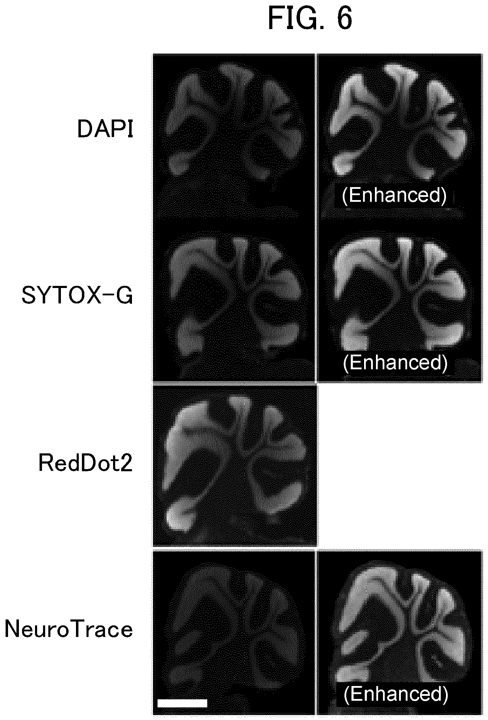

FIG. 6 is a diagram showing images of frozen sections prepared from mouse cerebellar hemisphere subjected to 3D staining with a dye in Example 3, wherein the scale bar corresponds to 1 mm;

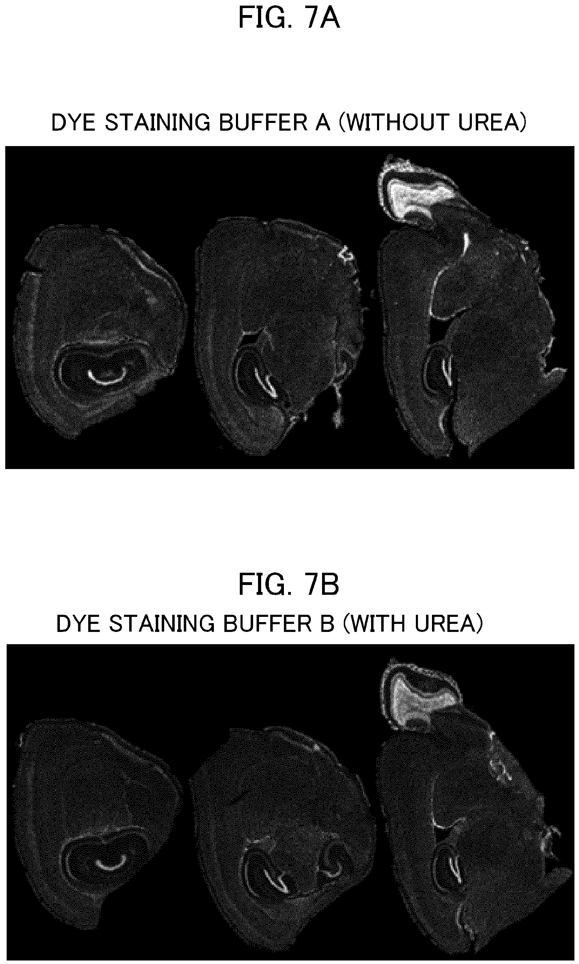

FIG. 7 is a diagram showing images of frozen sections prepared from mouse cerebral hemisphere subjected to 3D staining with a dye in Example 4, wherein FIG. 7A and FIG. 7B are diagrams showing images of frozen sections prepared from mouse cerebral hemisphere subjected to 3D staining with a urea-free dye staining buffer and a urea-containing dye staining buffer, respectively;

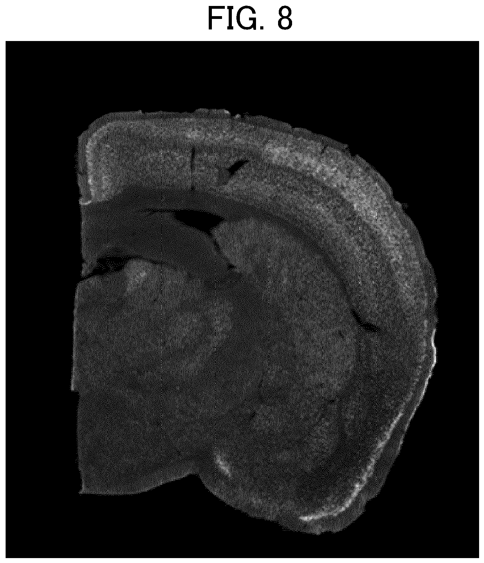

FIG. 8 is a diagram showing an image of a frozen section prepared from a piece of mouse cerebral hemisphere having a thickness of about 3 mm subjected to 3D immunostaining in Example 5;

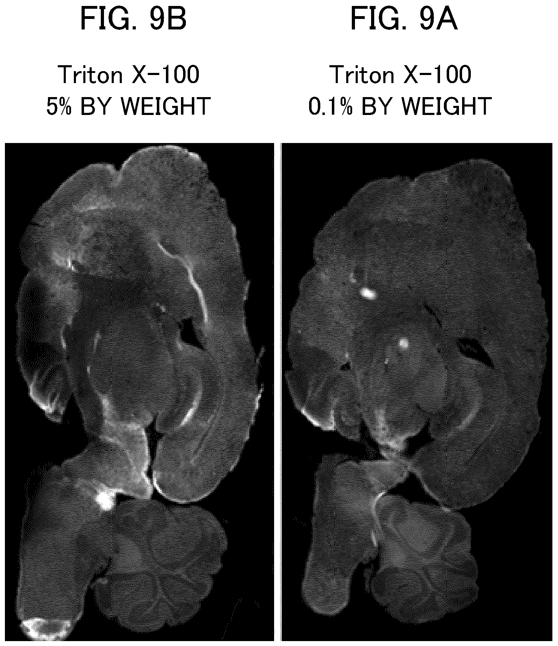

FIG. 9 is a diagram showing images of frozen sections of mouse brain subjected to immunostaining in Example 6, wherein FIG. 9A and FIG. 9B are diagrams showing images of frozen sections subjected to immunostaining with immunostaining buffers containing a nonionic surfactant at a concentration of 0.1% by weight or 5% by weight, respectively;

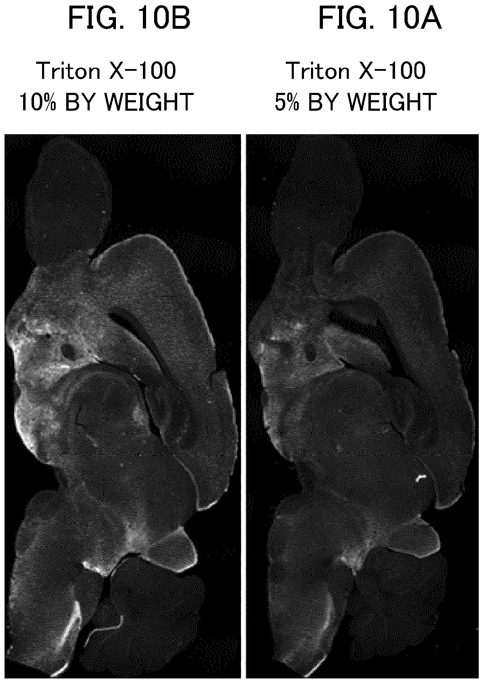

FIG. 10 is a diagram showing images of frozen sections of mouse brain subjected to immunostaining in Example 6, wherein FIG. 10A and FIG. 10B are diagrams showing images of frozen sections subjected to immunostaining with immunostaining buffers containing a nonionic surfactant at a concentration of 5% by weight or 10% by weight, respectively;

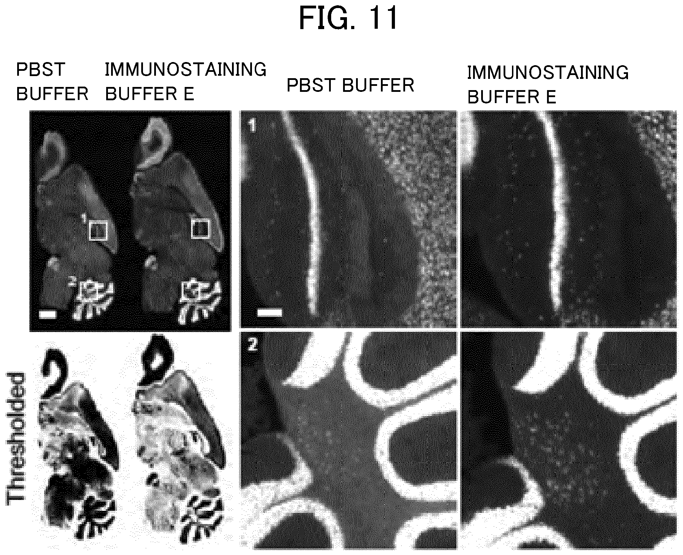

FIG. 11 is a diagram showing images of frozen sections of mouse brain subjected to immunostaining in Example 7, wherein the top left image shows whole sections in the sagittal direction; the top center panel and the top right panel show magnified images of the areas indicated with "1" in the top left image; the bottom center panel and the bottom right panel show magnified images of the areas indicated with "2" in the top left image; the scale bar in the image showing whole sections in the sagittal direction corresponds to 1 mm; and the scale bar in a magnified image corresponds to 100 µm;

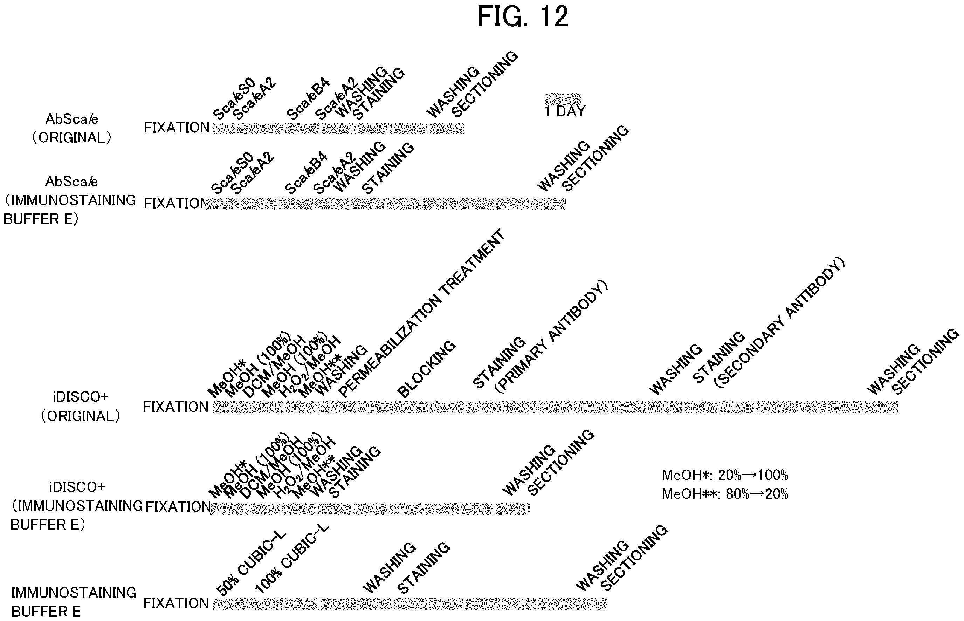

FIG. 12 is a diagram illustrating the steps of the 3D clearing-staining methods according to Example 8, wherein the steps are presented along the time axis;

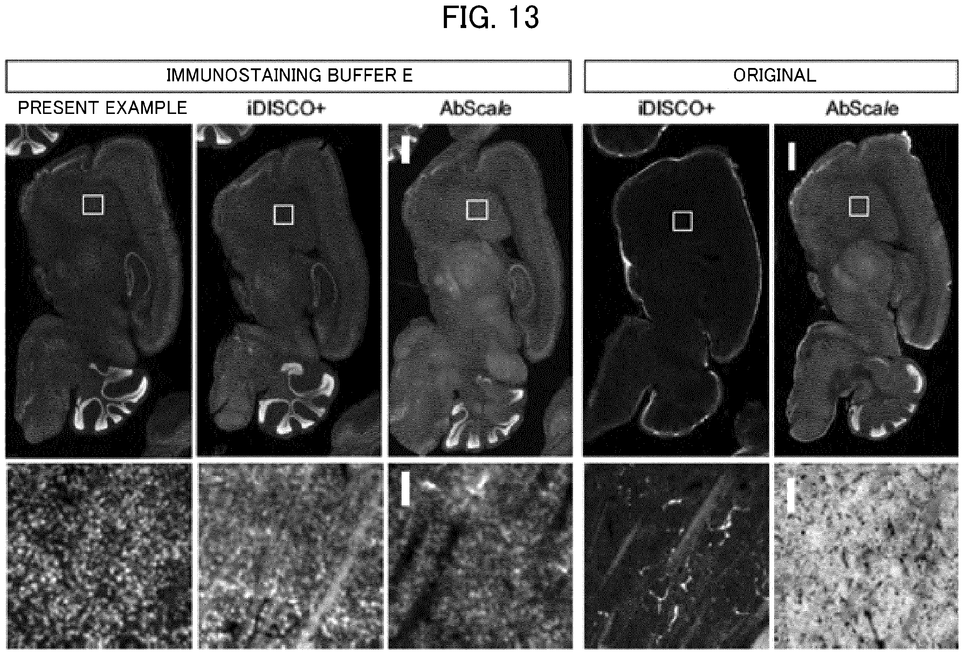

FIG. 13 is a diagram showing images of frozen sections prepared from mouse half brain subjected to 3D immunostaining based on the 3D clearing-staining methods shown in FIG. 12, wherein each scale bar in the images in the left column, which show whole sections in the sagittal direction, corresponds to 1 mm; and each scale bar in the magnified images in the right column corresponds to 50 µm;

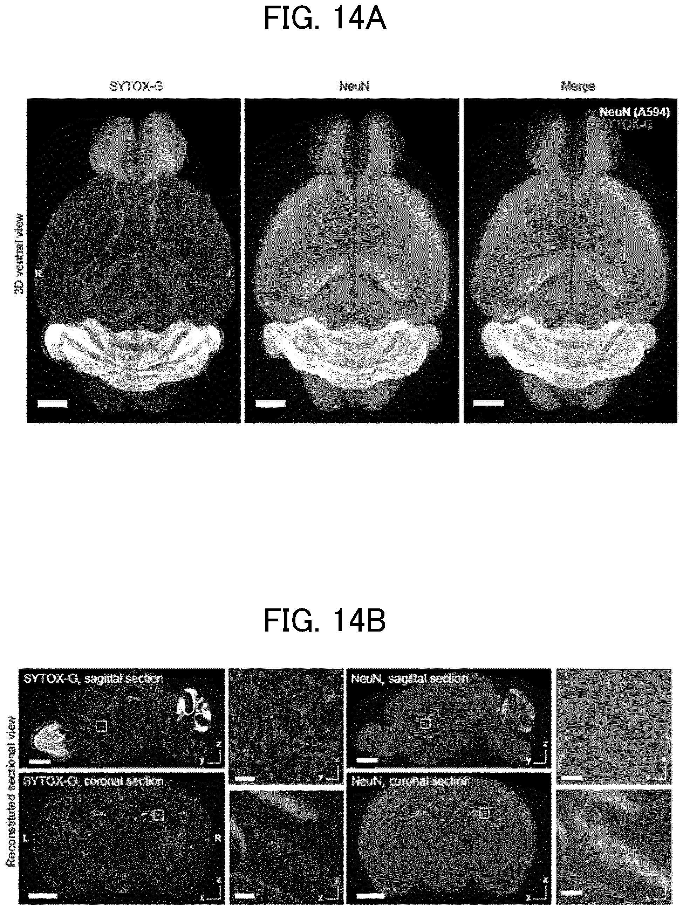

FIG. 14 is a diagram showing images of mouse whole brain subjected to 3D nuclear staining and 3D immunostaining in Example 9, wherein FIG. 14A is a diagram showing images of the signals of SYTOX (trademark)-Green (hereinafter referred to as "SYTOX-G") and NeuN, and an image prepared by merging these images; "L" represents "left", and "R" represents "right"; each scale bar in FIG. 14A corresponds to 2 mm; FIG. 14B is a diagram showing sagittal-plane and coronal-plane images of the signals of SYTOX-G and NeuN for the whole brain shown in FIG. 14A; and each scale bar in FIG. 14B corresponds to 100 µm;

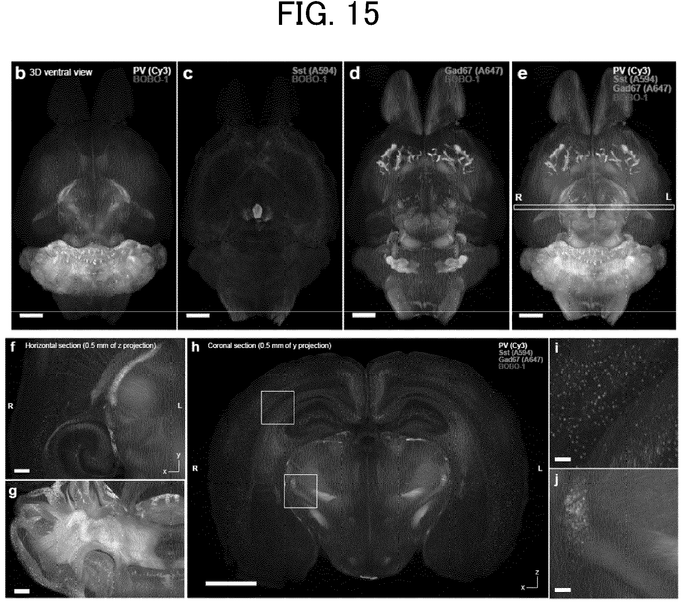

FIG. 15 is a diagram showing images of mouse whole brain subjected to 3D nuclear staining and 3D immunostaining in Example 10, wherein, together with the signal of BOBO (trademark)-1 iodide (462/481) (hereinafter referred to as "BOBO-1"), the signals of parvalbumin (PV), somatostatin (Sst), and glutamate decarboxylase (Gad) 67 are shown in panels b, c, and d, respectively, wherein each scale bar corresponds to 2 mm; the scale bar in panel e, which shows the signals of PV, Sst, and Gad67, corresponds to 2 mm; magnified images of part of the brain on a horizontal plane (x-y) are shown in panels f and g, wherein each scale bar corresponds to 0.5 mm; the scale bar in panel h, which shows a coronal-plane (x-z) image of an area shown in panel e, corresponds to 2 mm; and each scale bar in panels i and j, which show magnified images of areas shown in panel h, corresponds to 0.1 mm;

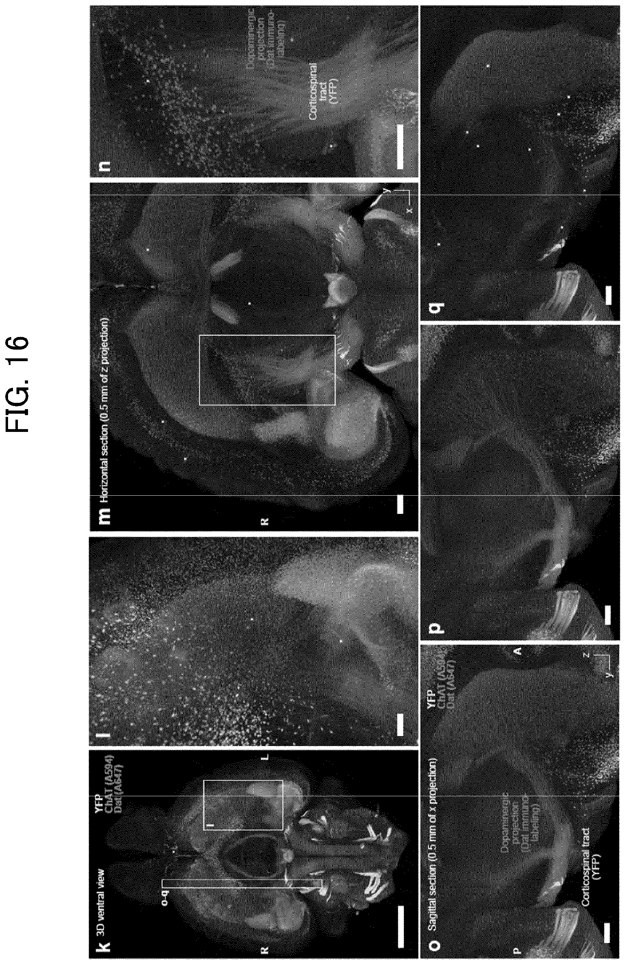

FIG. 16 is a diagram showing images of mouse whole brain subjected to 3D immunostaining in Example 11; wherein, together with the signal of yellow fluorescent protein (YFP), the signals of choline acetyltransferase (ChAT) and dopamine transporter (Dat) are shown in panel k, wherein the scale bar corresponds to 2 mm; the scale bar in panel 1, which shows a magnified image of the area indicated by "l" in panel k, corresponds to 0.5 mm; the scale bar in panel m, which shows an image of part of the brain on a horizontal plane, corresponds to 0.5 mm; the scale bar in panel n, which shows a magnified image of the area indicated by "n" in panel m, corresponds to 0.5 mm; and each scale bar in panels o, p, and q, which are reconstructed sagittal-plane images for the area indicated by "o-q" in panel k, corresponds to 0.5 mm;

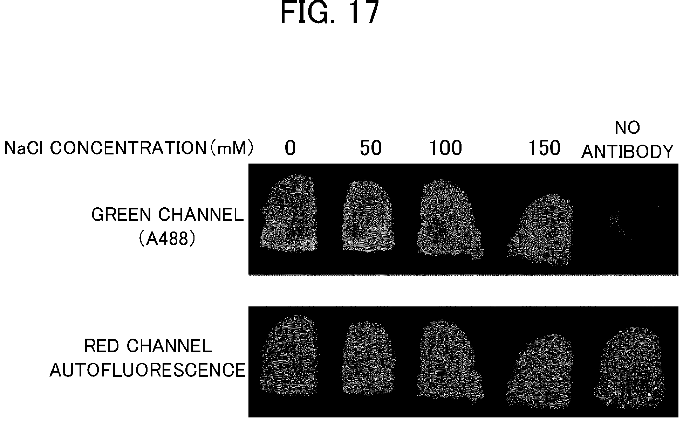

FIG. 17 is a diagram showing the signal of an antibody in mouse cerebellum according to Example 13;

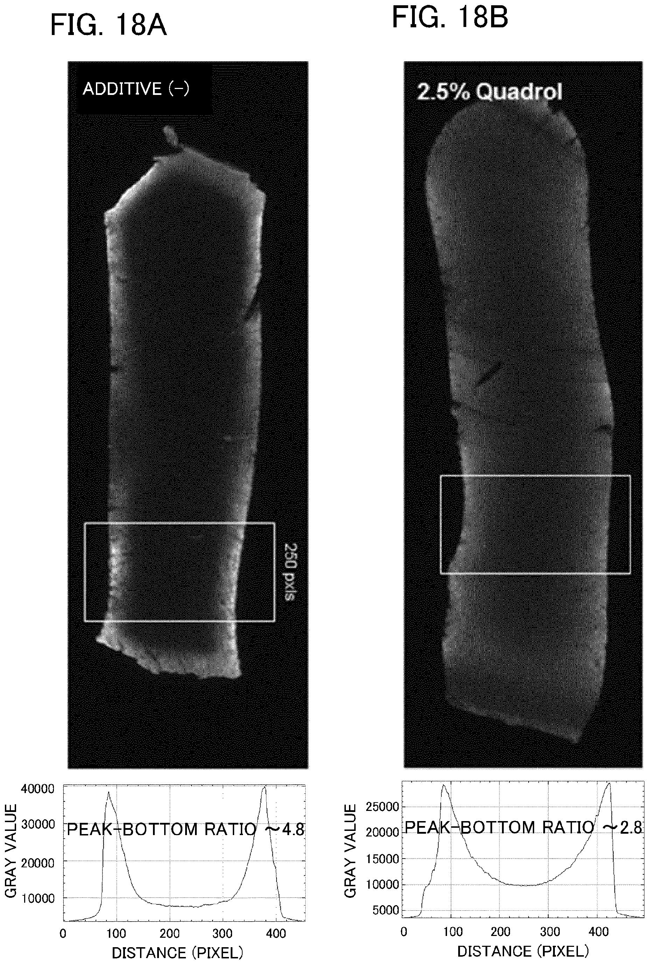

FIG. 18 shows images of frozen tissue sections of the cerebral cortex area of the pig according to Example 14, and their peak-bottom ratios, wherein FIG. 18A and FIG. 18B are diagrams for a case where immunostaining was carried out with an additive-free immunostaining buffer, and a case where immunostaining was carried out with an additive-containing immunostaining buffer, respectively;

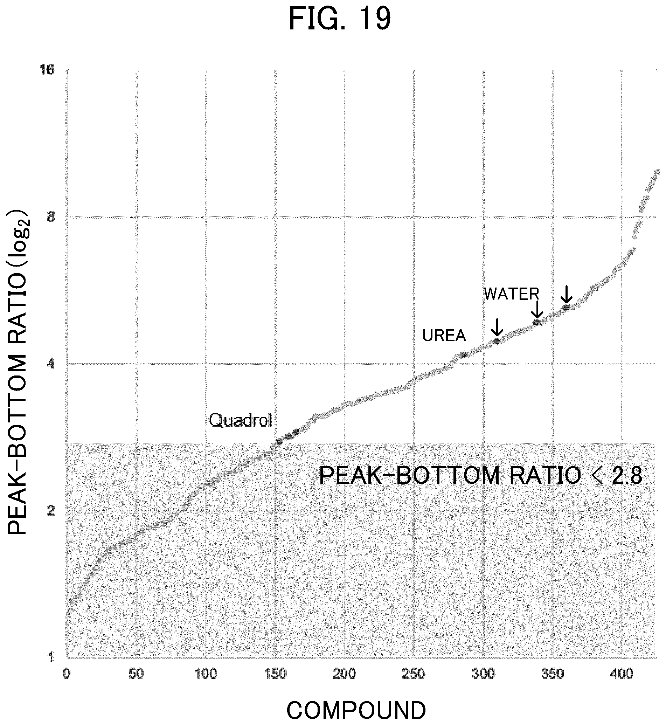

FIG. 19 is a diagram showing the peak-bottom ratios of the compounds according to Example 15;

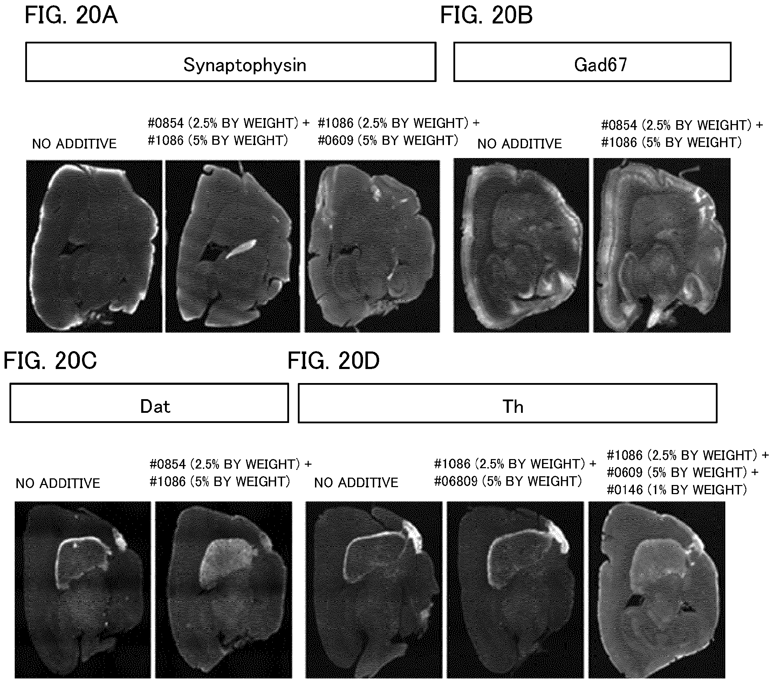

FIG. 20 is a diagram showing images of frozen sections prepared from mouse cerebral hemisphere subjected to 3D immunostaining in Example 16, wherein FIG. 20A, FIG. 20B, FIG. 20C, and FIG. 20D are diagrams showing images of frozen sections stained with antibodies against synaptophysin, Gad67, Dat, and Th, respectively;

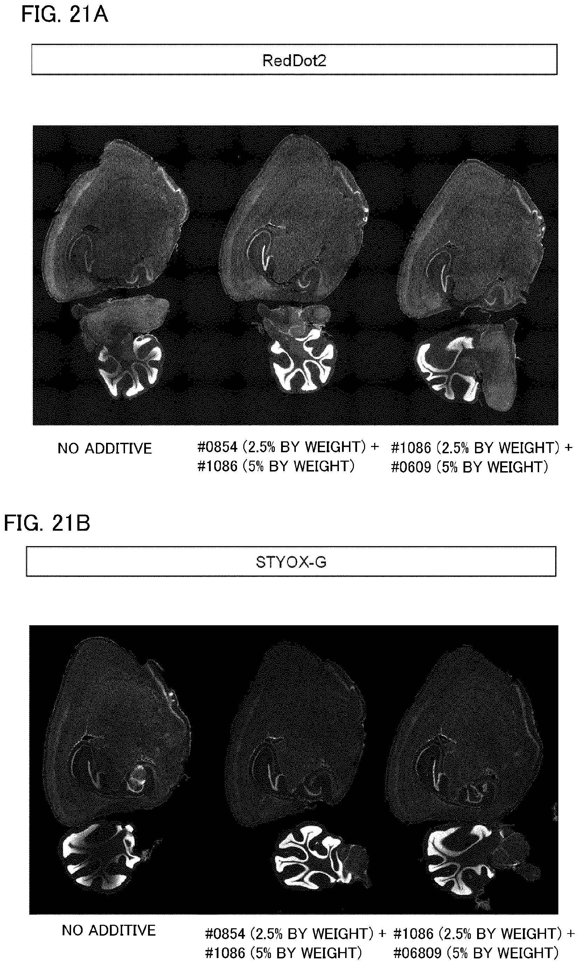

FIG. 21 is a diagram showing images of frozen sections prepared from mouse half brain subjected to 3D staining with a dye in Example 17 under conditions with no additives or with additives, wherein FIG. 21A and FIG. 21B are diagrams showing images of the signals of RedDot2 and SYTOX-G, respectively;

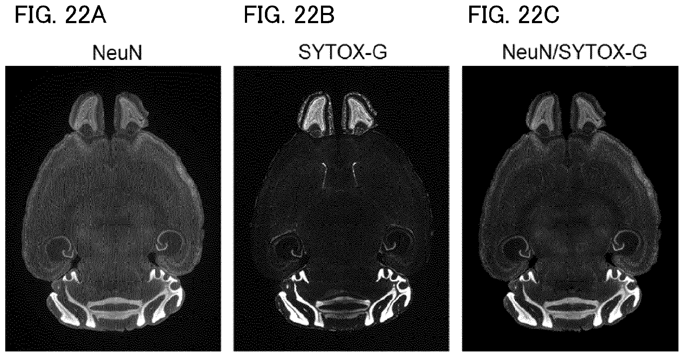

FIG. 22 is a diagram showing images of frozen sections prepared from mouse cerebral hemisphere subjected to 3D staining with a dye and an antibody in Example 18; wherein FIG. 22A is a diagram showing an image of the signal of NeuN; FIG. 22B is a diagram showing an image of the signal of SYTOX-G; and FIG. 22C is a diagram showing an image prepared by merging the image showing the signal of NeuN and the image showing the signal of SYTOX-G;

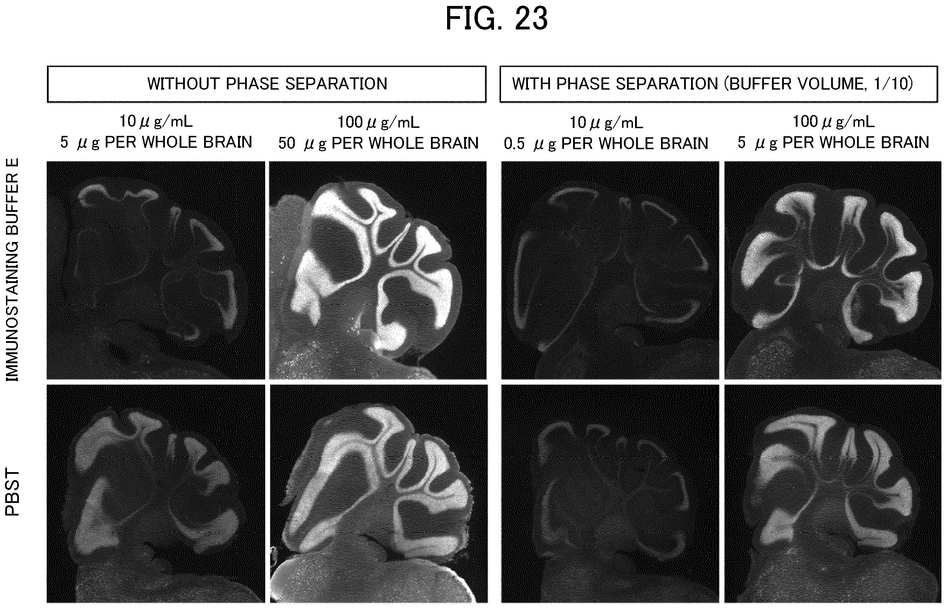

FIG. 23 is a diagram showing images of frozen sections prepared from mouse cerebellar hemisphere subjected to 3D immunostaining in Example 19 under conditions without phase separation or with phase separation; and

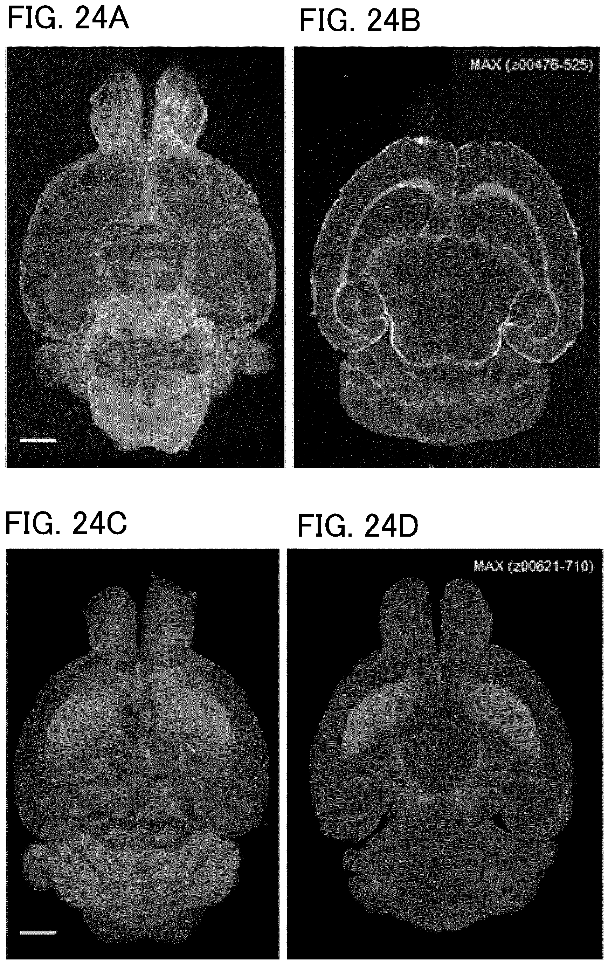

FIG. 24 is a diagram showing images of mouse whole brain subjected to 3D nuclear staining and 3D immunostaining at the same time in Example 20, wherein FIG. 24A is a diagram showing three-dimensional reconstructed images showing the signals of GFAP and SYTOX-G; FIG. 24B is a diagram showing an image of the signal of GFAP; FIG. 24C is a diagram showing a three-dimensional reconstructed image showing the signals of Dat and SYTOX-G; and FIG. 24D is a diagram showing an image of the signal of Dat.

### Description of Embodiments

Embodiments according to the present disclosure are described below. The present disclosure is not limited by the embodiments described below. Unless otherwise specified in the present description, "%" means % by weight.

### Embodiment 1

A biological tissue-staining reagent according to the present embodiment is a reagent suitable for staining of a biological tissue. The biological tissue-staining reagent includes a nonionic surfactant and a salt. More specifically, the biological tissue-staining reagent may be a solution, preferably a buffer, containing the nonionic surfactant and the salt described above. The main solvent of the solution or the buffer is, for example, water. Water may be used alone as the solvent.

Examples of the nonionic surfactant include fatty acid-based surfactants, higher-alcohol-based surfactants, and alkylphenol-based surfactants. Examples of the fatty acid-based surfactants include polyoxyethylene sorbitan monolaurate, polyoxyethylene sorbitan monopalmitate, polyoxyethylene sorbitan monostearate, and polyoxyethylene sorbitan monooleate. Examples of the higher-alcohol-based surfactants include polyvinyl alcohol. Examples of the alkylphenol-based surfactants include polyoxyethylene octylphenyl ether.

The nonionic surfactant is preferably at least one selected from the group consisting of the Triton X (trademark) series such as Triton X-100 and Triton X-140; the Tween (trademark) series such as Tween-20, Tween-40, Tween-60, and Tween-80; and NP-40 (trade name). When necessary, a mixture of two or more nonionic surfactants may be used.

The nonionic surfactant in the biological tissue-staining reagent according to the present embodiment is at a concentration higher than 1%. The concentration of the nonionic surfactant in the biological tissue-staining reagent is, for example, 2 to 30%, 3 to 20%, 4 to 15%, or 5 to 12%. The concentration of the nonionic surfactant in the biological tissue-staining reagent is preferably 5% or 10%.

The salt is a compound in which an anion derived from an acid is ionically bonded to a cation derived from a base. The salt may be a compound produced by neutralization reaction between an acid and a base, and may be a compound in which a hydrogen ion of an acid is substituted with a metal. The salt is preferably a normal salt, for example, sodium chloride (NaCl), calcium chloride (CaCl₂), lithium chloride (LiCl), and potassium chloride (KCl), whose aqueous solution is neutral.

The concentration of the salt in the biological tissue-staining reagent according to the present embodiment is not less than 200 mM. The concentration of the salt is, for example, 200 to 2000 mM, 200 to 1500 mM, 200 to 1000 mM, 200 to 800 mM, 200 to 600 mM, or 200 to 500 mM. The concentration of the salt in the biological tissue-staining reagent is preferably 200 mM or 500 mM.

The biological tissue-staining reagent according to the present embodiment may be used both in cases where the biological tissue is stained with a dye, and in cases where the biological tissue is stained with an antibody through immune reaction. As the dye for staining the biological tissue, a known dye used for staining biological tissues may be suitably used. The dye is preferably a small-molecule compound or a low molecular weight compound that binds to a particular molecule or the like constituting the biological tissue. The dye may be, for example, a dye that binds to nucleic acid to achieve staining of a tissue. Examples of the dye include PI, SYTO 16, DAPI, SYTOX-G, BOBO-1, RedDot (trademark) 2 Far-Red Nuclear Stain (hereinafter referred to as "RedDot2"), and NeuroTrace (trademark) Fluorescent Nissl Stain (hereinafter referred to as "NeuroTrace"). The dye may be, for example, a nuclear dye or a nucleic acid dye.

In cases where the biological tissue-staining reagent is used for immunostaining, the antibody is, for example, an immunostaining antibody; or immunoglobulin derived from a culture supernatant of a hybridoma, from an ascites, antiserum, or antiplasma of an animal subjected to intrasplenic immunization, or from serum of a bird egg. In cases where a biological tissue is stained with an antibody, the biological tissue-staining reagent is preferably used for one-step immunostaining using a primary antibody labeled with a dye such as a fluorescent dye, or using a complex of a primary antibody and a Fab fragment antibody labeled with a dye. Table 1 and Table 2 below show preferred examples of the antibody. Examples of the dye for labeling the antibody include dyes such as Alexa Fluor (trademark) dye, fluorescein isothiocyanate (FITC), and Cy dye.

In cases where the biological tissue-staining reagent is used for immunostaining, the biological tissue-staining reagent according to the present embodiment preferably further includes a neutral buffer. The neutral buffer means a buffer having a pH of 6 to 8, preferably 7.2 to 7.8, more preferably 7.4 to 7.6, especially preferably 7.5. Specific examples of the neutral buffer include phosphate buffer, Tris buffer, HEPES buffer, acetate buffer, carbonate buffer, and citrate buffer. PBS, D-PBS, Tris-buffered saline, and HEPES-buffered saline, which are physiological salines of those buffers, may also be used as the neutral buffer. The neutral buffer is especially preferably HEPES buffer, and its concentration is 5 to 30 mM or 8 to 20 mM, preferably 8 to 12 mM or 10mM.

In cases where the biological tissue-staining reagent is used for immunostaining, the biological tissue-staining reagent preferably further includes a blocking reagent. The blocking reagent is not limited as long as the reagent is for use in prevention of non-specific binding of antibody. Examples of the blocking reagent include skim milk, bovine serum albumin (BSA), fish gelatin, horse serum, fetal bovine serum (FBS), and casein. The blocking reagent is preferably casein. The concentration of the blocking reagent in the biological tissue-staining reagent is appropriately set. The concentration is, for example, 0.1 to 3%, 0.2 to 2%, 0.3 to 1%, or 0.4 to 0.8%, preferably 0.5%.

The biological tissue-staining reagent according to the present embodiment may also contain, in addition to the above components, an additive that promotes penetration of the dye or the antibody into the biological tissue. The additive is, for example, a compound that promotes penetration of the dye or the antibody into the biological tissue. As described in the Examples below, the compound may be selected by evaluating the penetration efficiency of the dye or the antibody into the biological tissue in staining of the biological tissue with the dye or the antibody.

Examples of the additive include compounds of aromatic amines, aliphatic amides, nicotinamides, sulfamides, sulfonic acid salts, amino alcohols, alcohols, sulfinic acids, thioureas, or carboxylic acids represented by General Formula (1) below except urea and urea derivatives. In General Formula (1), R₁, R₂, R₃, and R₄ are each independently a hydrogen atom, a halogen atom, or a hydrocarbon group, wherein, in a case where a plurality of carbon atoms constitutes the hydrocarbon group, part of the carbon atoms are optionally substituted by a heteroatom such as a nitrogen atom, an oxygen atom, or a sulfur atom. The hydrocarbon group includes chain hydrocarbon groups and cyclic hydrocarbon groups.

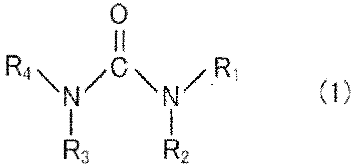

The additive may also be a compound containing a cyclic amine, cyclic amide, chain amine, chain amide, or sulfo group represented by General Formula (1) except urea and urea derivatives, or a combination thereof.

The additive is preferably an amino alcohol. Examples of the amino alcohol include N,N,N',N'-tetrakis(2-hydroxypropyl)ethylenediamine (hereinafter referred to as "Quadrol"), triethanolamine, triisopropanolamine, 2-amino-1,3-propanediol, 3-methylamino-1,2-propanediol, 3-amino-1,2-propanediol, N,N,N,N-tetrakis(2-hydroxyethyl)ethylenediamine, and N-butyldiethanolamine. In cases where the biological tissue to be stained contains a fluorescent protein, the amino alcohol is especially preferably Quadrol, triethanolamine, triisopropanolamine, 2-amino-1,3-propanediol, and N-butyldiethanolamine.

More specific examples of the additive are selected from the group consisting of pyridazine, 2-cyanoacetamide, 5-methyl-2-pyrrolidone, methacrylamide, nicotinamide, N,N-bis(2-cyanoethyl)formamide, nicotinic acid hydrazide, 4-cyanopyridine, butylamide, 4-methylpyridine N-oxide, isonicotinamide, 1,5-pentamethylenetetrazol, 2,5-dimethylpyrazine, thiomorpholine, 2-pyrrolidone, 2-pyridinecarboxylic acid hydrazide, 4-(2-aminoethyl)morpholine, sodium o-sulfobenzimide dihydrate, 4-acryloylmorpholine, ε-caprolactam, methyl carbamate, glutaronitrile, 4-acetamide cyclohexanol, 2-[2-(dimethylamino)ethoxy]ethanol, 1,3-bis[tris(hydroxymethyl)methylamino]propane, 2-picolinamide, sodium 4-chlorobenzenesulfonate, sodium dehydrocholate, sodium 4-chlorobenzenesulfinate, N,N-diethylpropionamide, 2-piperidone, 2,2,2-trifluoroethanol, N,N-diethylnicotinamide, 1,3-dimethylthiourea, N-methylnicotinamide, 3-dimethylaminopropionitrile, N,N-dimethylmethacrylamide, tetrahydrothiophene 1,1-dioxide, nicotine, sodium 2,5-dichlorosulfanilate, benzyl benzoate, sodium 2,4-dimethylbenzenesulfonate monohydrate, acetoxime, diacetone acrylamide, 1,2-cyclohexanediol, 3-picolylamine, 2,3-dimethyl-1-phenyl-5-pyrazolone, N,N,N',N'-tetramethylethylenediamine, sodium hippurate, N-ethylsuccinimide, propionamide, 1-[2-(2-hydroxyethoxy)ethyl]piperazine, 3-ethyl-3-oxetanemethanol, N,N-dimethylethylamine, 2-aminopyrazine, morpholine, 1,2-bis(2-aminoethoxy)ethane, 1-butyl-4-methylpyridinium chloride, 3-acetamide piperidine, N,N-diethylformamide, N,N-dimethylethylenediamine, 2-(2-aminoethoxy)ethanol, ethyl N-methylcarbamate, 2-methylthiazole, disodium diphenylsulfone-4,4'-dichloro-3,3'-disulfonate, 1-(3-aminopropyl)imidazole, sodium heptanoate, N,N-dimethylacrylamide, 2,2'-diamino-N-methyldiethylamine, tetrahydrofurfurylamine, 3-hydroxy-1-methylpiperidine, methylglyoxime, 1-(2-hydroxyethyl)-4-(3-hydroxypropyl)piperidine, dimethylsulfone, dimethylacetamide, 1-amino-2-butanol, DL-2-amino-1-butanol, 3-sulfopropyl methacrylate potassium salt, pyrazine, N-methylformamide, 4-hydroxy-2-butanone, N,N-diethylacrylamide, 1,4-benzenedimethanol, sodium quinaldinate, 2-amino-2-methyl-1,3-propanediol, 2-methylimidazole, 2,4-diaminopyrimidine, sodium n-octanoate, N-methylthiourea, ethanol, sodium 1-naphthaleneacetate, 2-ethylimidazole, sodium benzenesulfinate dihydrate, N,N,N',N",N"-pentamethyldiethylenetriamine, bis(2-hydroxyethyl)aminotris(hydroxymethyl)methane, 1-butyl-4-methylpyridinium bromide, 4-formylmorpholine, triethylenetetramine, 3-amino-5-methylthio-1H-1,2,4-triazole, sodium pyridine-3-sulfonate, sodium 2,4-dichlorophenoxyacetate monohydrate, 1-(2-dimethylaminoethyl)-4-methylpiperazine, sodium 1-pentanesulfonate, N-nitrosodiethylamine, isonipecotamide, hydrazine monohydrate, sodium 2-naphthalenesulfonate, maltitol, sodium 1-butanesulfonate, 4-(4-hydroxybutyl)thiomorpholine 1,1-dioxide, 4-tert-butyl-1-(3-sulfopropyl)pyridinium hydroxide inner salt hydrate, sodium benzenesulfonate, 2,6-pyridinedimethanol, N,N-diethylisonicotinamide, veratryl alcohol, N,N'-diacetylethylenediamine, imidazole, sodium 4-ethylbenzenesulfonate, sodium 2-sulfobenzaldehyde, 1-butanol, tetrahydrofuran, adipic acid dihydrazide, tetrabutylammonium bromide, 3,3'-iminodipropionitrile, sulfamide, bis(2-methoxyethyl)amine, ethylene cyanohydrin, piperazine hexahydrate, lactamide, N,N-dimethylbenzamide, N,N,N' ,N" ,N" -pentakis(2-hydroxypropyl)diethylenetriamine, sodium p-toluenesulfonate, 2-cyclohexylaminoethane sulfonic acid, 1,6-hexanediol, N-tert-butyl-2-methoxyethylamine, N-methylacetamide, acetamide, 1-methylpiperazine, 1-methylpyridinium chloride, 1-(2-pyrimidyl)piperazine, 2-hydroxyethylmethylsulfone, ethylene cyanohydrin, chlorocholine chloride, benzyltrimethylammonium bromide, 2-(2-aminoethylamino)ethanol, N-isopropylacrylamide, sodium isonicotinate, amylamine, 1-amino-2-propanol, ammonium (+)-3-bromocamphor-8-sulfonate, 3-amino-1,2-propanediol, N-methyldiethanolamine, and isopropanolamine phosphate.

The four compounds in FIG. 1A are representative examples of the additives having neutral pHs. The two compounds in FIG. 1B are representative examples of the additives having alkaline pHs. A combination of a plurality of compounds may be provided as the additive. One or more of the above compounds may be arbitrarily combined to provide the additive. The following are combinations preferred as the additive: Combination 1 of nicotinic acid hydrazide (#0854), which is shown in FIG. 1A, and pyrazine (#1086); Combination 2 ofN,N-diethylnicotinamide (#0609) and pyrazine (#1086); Combination 3 of nicotinic acid hydrazide (#0854), pyrazine (#1086), and 2-[2-(dimethylamino)ethoxy]ethanol (#0146), which is shown in FIG. 1B; and Combination 4 ofN,N-diethylnicotinamide (#0609), pyrazine (#1086), and 2-[2-(dimethylamino)ethoxy]ethanol (#0146).

The concentration of the additive in the biological tissue-staining reagent is not limited, and is, for example, 0.1 to 10% by weight, 0.5 to 8% by weight, 1 to 7% by weight, 2 to 6% by weight, or 2.5 to 5% by weight. In cases where the additive contains one or more of the above compounds as components, the concentration of each component may be 0.1 to 10% by weight, 0.5 to 8% by weight, 1 to 7% by weight, 2 to 6% by weight, or 2.5 to 5% by weight, and the concentrations of the components in the biological tissue-staining reagent may be either the same or different.

The biological tissue-staining reagent according to the present embodiment may contain the dye described above. The concentration of the dye in the biological tissue-staining reagent is not limited, and is set in accordance with the volume and the type of the biological tissue to be stained, the experimental conditions, and the like. The concentration of the dye is, for example, 1 to 10 µg/mL, 2 to 8 µg/mL, or 3 to 5 µg/mL.

The biological tissue-staining reagent according to the present embodiment may contain the antibody described above. The concentration of the antibody in the biological tissue-staining reagent is not limited, and is set in accordance with the volume and the type of the biological tissue to be stained, the experimental conditions, and the like. The concentration of the antibody is, for example, 0.05 to 50 µg/mL, preferably 3 to 40 µg/mL, especially preferably 5 to 20 µg/mL.

The biological tissue to be stained with the reagent is, for example, a sample derived from an animal, or a sample derived from a plant. Examples of the animal include animals such as fish, amphibians, reptiles, birds, and mammals. The biological tissue is preferably a biological tissue of a mammal. Examples of the mammal include, but are not limited to, mice, rats, rabbits, guinea pigs, marmosets, dogs, cats, ferrets, pigs, cows, horses, monkeys and chimpanzees, and humans.

The biological tissue may be an individual itself except living humans, or may be an organ, a tissue, a cell cluster, or a cell obtained from an individual of a multicellular organism. Preferably, the biological tissue is, for example, a whole brain, or part of a brain such as a cerebral hemisphere.

The biological tissue may be a sample subjected to fixation treatment for observation under the microscope. The biological tissue is preferably fixed by a known method using formaldehyde (FA), paraformaldehyde (PFA), or the like. The fixation treatment is preferably followed by, for example, treatment by soaking in phosphate-buffered saline (PBS).

The biological tissue may be, for example, a biological tissue in which a fluorescent chemical substance is injected, a biological tissue stained with a fluorescent chemical substance, a biological tissue in which a cell expressing a fluorescent protein is transplanted, and a biological tissue of a genetically modified animal expressing a fluorescent protein.

A method of using the biological tissue-staining reagent according to the present embodiment is described below. The biological tissue-staining reagent herein preliminarily contains a dye. A sample fixed and defatted as described above as the biological tissue is stained with a dye. For the defatting of the sample, for example, the sample may be soaked in CUBIC-L (water containing 10% by weight N-butyldiethanolamine and 10% by weight Triton X-100), CUBIC-1 (water containing 15% by weight Triton X-100, 25% by weight Quadrol, and 25% by weight urea), or CUBIC-1A (water containing 10% by weight Triton X-100, 5% by weight Quadrol, 10% by weight urea, and 25 mM NaCl) at 37°C for several days to several weeks.

In the staining step, in which the sample is stained with the dye, the sample is exposed to the biological tissue-staining reagent. The length of time of the exposure is not limited as long as the biological tissue-staining reagent penetrates into the inside of the sample. For example, in the staining step, the sample is soaked in the biological tissue-staining reagent at 37°C for 2 to 5 days. After the defatting of the sample, the sample is preferably washed with PBS or the like before carrying out the staining step. After the staining step, the sample may be washed with PBS or the like.

Whether or not the sample is stained can be confirmed by a known method using an optical microscope or the like capable of detecting the dye. In cases where the dye is to be detected using a fluorescence microscope or the like, a frozen section may be prepared from the sample by a known method, and the frozen section may be observed using an optical microscope or the like. The observation of the sample may be carried out using any type of optical microscope. For example, the sample may be observed by three-dimensional super-resolution microscopy (such as STED, 3D PALM, FPALM, 3D STORM, and SIM). The sample may also be observed by application of multiphoton excitation optical microscopy. The sample may also be observed using a single-photon confocal microscope or a light-sheet fluorescence microscope(LSFM).

A method of immunostaining using the biological tissue-staining reagent according to the present embodiment is described below. The biological tissue-staining reagent herein preliminarily contains an antibody. In the immunostaining step, in which the sample is stained with the antibody, the sample is exposed to the biological tissue-staining reagent similarly to the staining step. For example, in the immunostaining step, the sample is soaked in the biological tissue-staining reagent at 23 to 37°C or 28 to 34°C, preferably at 32°C. The length of time of the exposure in the immunostaining step is not limited as long as the biological tissue-staining reagent penetrates into the inside of the sample. The length of time may be 1 day to 8 weeks, 2 days to 7 weeks, 3 days to 6 weeks, 4 days to 5 weeks, or 1 to 4 weeks.

With the biological tissue-staining reagent according to the present embodiment, staining using a dye and immunostaining using an antibody can be carried out for the same sample. FIG. 2 shows examples of the steps constituting biological tissue-staining methods in cases where nuclear staining and immunostaining are carried out for a sample that is a mouse whole brain, wherein each step is presented together with the required time and the temperature. The biological tissue-staining method shown in FIG. 2A includes: a fixation step of fixing the sample; a defatting step of defatting the sample; a nuclear staining step of staining the nuclei of the sample; an immunostaining step; a postfixation step; and a refractive index (RI) adjustment step. In the RI adjustment step, the RI of the sample is adjusted to achieve clearing that allows three-dimensional observation of the sample using an optical microscope. In the RI adjustment step, the sample may be embedded in a gel or the like, when necessary. In the biological tissue-staining method, a washing step of washing the sample with PBS or the like is preferably carried out between the steps.

The biological tissue-staining method exemplified in FIG. 2B includes an enzyme treatment step of treating the sample with an enzyme, between the nuclear staining step and the immunostaining step. In the enzyme treatment step, the sample is partially digested with hyaluronidase, collagenase-P, or the like to suppress binding of the antibody to sample edges, to thereby promote penetration of the antibody to the inside.

The biological tissue-staining method exemplified in FIG. 2C includes a pretreatment step of exposing the defatted sample to a slightly acidic buffer containing an antibody, between the nuclear staining step and the immunostaining step. The defatted sample has chemical properties similar to an anionically charged electrolyte gel. Therefore, when the sample and the antibody are kept under slightly acidic conditions, a shift toward the acidic side occurs relative to the isoelectric point, so that the cationically charged antibody forms a reversible complex with the sample due to electrical interaction (see Example 13 below). Thus, concentration and penetrability of the antibody in the sample can be improved.

As the slightly acidic buffer, a known arbitrary buffer may be used. The pH of the slightly acidic buffer is, for example, 4 to 6, 4.5 to 5.5, or 4.8 to 5.2. The pH of the slightly acidic buffer is preferably 5. More specifically, the slightly acidic buffer is, for example, citrate buffer and acetate buffer. The slightly acidic buffer may contain a salt at a predetermined concentration such 100 to 500 mM, 100 to 300 mM, or 100 to 200 mM. The slightly acidic buffer may also contain, for example, a compound that promotes penetration of the antibody to the biological tissue.

In the biological tissue-staining method according to the present embodiment, the enzyme treatment step and the pretreatment step may be used in combination. In such a case, for example, the enzyme treatment step may be carried out followed by washing of the sample, and then the pretreatment step may be carried out.

As shown in Example 11, the biological tissue-staining reagent according to the present embodiment enables 3D staining of even a biological tissue containing a fluorescent protein, without fading of the fluorescent protein. Thus, the biological tissue-staining reagent is applicable to a wide range of biological tissues. Further, as shown in Examples 1 to 4 below, the biological tissue-staining reagent enables sufficient and uniform penetration of a variety of dyes into defatted biological tissues. Thus, targets in biological tissues can be more securely labeled.

Further, as shown in Examples 5, 6, and 9 below, the biological tissue-staining reagent according to the present embodiment even enables sufficient and uniform penetration of antibodies into defatted biological tissues. Further, with the biological tissue-staining reagent according to the present embodiment, a good signal-background ratio (SBR) can be obtained as shown in Example 7 below. Therefore, more accurate information can be obtained in, for example, image analysis of a stained biological tissue.

As shown in Example 8, the biological tissue-staining reagent according to the present embodiment can be incorporated in an existing 3D clearing-staining method, and enables penetration of an antibody to the inside of the sample. As shown in Examples 9 and 10 below, even in cases of combined use of a dye and an antibody, or of a plurality of kinds of antibodies in the same sample, the biological tissue-staining reagent is capable of securely labeling targets without crossing. Further, the biological tissue-staining reagent is applicable to many kinds of antibodies as shown in Example 12 below.

The biological tissue-staining method according to the present embodiment may include a pretreatment step of exposing a defatted biological tissue to a slightly acidic buffer containing an antibody. Therefore, concentration of the antibody in the tissue and penetration of the antibody into the tissue can be improved, so that the target in the tissue can be securely labeled. Of course, as shown in FIG. 2A and FIG. 2B, the biological tissue-staining method enables secure labeling of the target in the tissue even in cases where the immunostaining step is carried out without carrying out the pretreatment step after the nuclear staining step.

The biological tissue-staining reagent and the biological tissue-staining method according to the present embodiment are applicable not only to 3D staining, but also to the so-called 2D staining, in which tissue sections are stained.

Further, the biological tissue-staining reagent and the biological tissue-staining method according to the present embodiment enable sufficient and uniform penetration of dyes and antibodies into defatted biological tissues. Thus, by staining markers related to nerves, such as c-Fos, functional analysis of neural circuits and neuronal responses is possible at a cellular-level resolution.

Further, by inclusion of the additives described above in the biological tissue-staining reagent according to the present embodiment, penetration of dyes and antibodies into a sample can be promoted. Therefore, the staining performance inside the sample can be enhanced, so that the whole sample can be uniformly stained (see mainly Examples 16 to 18, and 20 below). The additives described above promote penetration of dyes and antibodies into a sample not only in cases where the sample is stained with the biological tissue-staining reagent according to the present embodiment, but also in cases where the sample is stained with a conventional staining buffer.

In another embodiment, a biological tissue-staining kit is provided. The biological tissue-staining kit includes: the biological tissue-staining reagent described above; and a dye. The biological tissue-staining kit may include: the biological tissue-staining reagent described above; and an antibody. In this case, the biological tissue-staining kit may further include the slightly acidic buffer described above. The biological tissue-staining kit may further include a washing buffer containing: a nonionic surfactant at a concentration higher than 1%; a salt at not less than 200 mM; and a neutral buffer. The washing buffer is suitable for washing of the sample after immunostaining. After the immunostaining step, the sample may be stored at 4°C overnight, and may then be washed with the washing buffer.

The biological tissue-staining kit may further include an instruction or a direction. The instruction or direction includes, for example, description of the composition of the biological tissue-staining reagent or a protocol related to the biological tissue-staining method. The biological tissue-staining reagent may further include additives other than the components described above, such as a pH adjusting agent, an osmoregulating agent, an antiseptic, and an agent for suppressing dryness of a sample. The additives may be contained in the biological tissue-staining kit separately from the biological tissue-staining reagent.

The biological tissue-staining kit is a package including a container including a particular material such as a component. In the biological tissue-staining kit, a plurality of constituents thereof may be contained as a mixture in the same container, or may be contained in separate containers. The instruction or direction may be recorded in a recording medium such as paper or magnetic tape, or such as an electronic medium including a computer-readable disk, tape, or CD-ROM. The biological tissue-staining kit may include a container containing a diluent, solvent, washing liquid, or another reagent. Further, the biological tissue-staining kit may include an instrument and a reagent for carrying out a procedure for realizing application of the kit.

Specific examples of configuration of the biological tissue-staining kit include the following.

### [Staining Kit for Dye]

### (Configuration)

1. Dye staining buffer, for n times of use (4 mL / mouse whole brain)
2. Recording medium in which a protocol is recorded

### (Specification)

Dye 3D staining buffer: 10% Triton X-100, 5% Quadrol, and 500 mM NaCl

### [Staining Kit for Immunostaining]

### (Configuration)

1. Immunostaining buffer (1 × or 2× concentration), for n times of use (15.5 mL / mouse whole brain at 1 × concentration)
2. Additive for immunostaining (10× concentration), for n times of use (maximum, 100 µL (final 2× / mouse whole brain))
3. Tube for staining
4. Immunostaining washing buffer, for n times of use (30 mL (15 mL ×2 times of use) / mouse whole brain)
5. Agent for fixation after immunostaining, for n times of use (0.2 mL / mouse whole brain)
6. Recording medium in which a protocol is recorded

### (Specification)

Immunostaining buffer: (1 ×) 10 mM HEPES (pH 7.5), 10% Triton X-100, 200 mM NaCl, 0.5% casein
Additive for immunostaining: (10×) 25% Quadrol
Tube for staining: 15-mL tube for staining
Immunostaining washing buffer: 10 mM HEPES (pH 7.5), 10% Triton X-100, and 500 mM NaCl
Agent for fixation after immunostaining: saturated FA (37 to 38%) / methanol*
   *The agent for fixation after immunostaining was used after dilution to 1% in the immunostaining washing buffer in the tube for staining.

### Embodiment 2

Staining with a dye and immunostaining with an antibody can be carried out simultaneously for the same biological tissue by using the appropriate additives described above depending on the dye and the antibody. A biological tissue-staining method according to the present embodiment is described below mainly regarding the difference from Embodiment 1. The biological tissue-staining method according to the present embodiment includes a simultaneous staining step of exposing a sample to a biological tissue-staining reagent including: a dye; an immunostaining antibody; and an additive. Similarly to the staining step, the sample is exposed to the biological tissue-staining reagent. For example, in the simultaneous staining step, the sample is soaked in the biological tissue-staining reagent at 23 to 37°C or 28 to 34°C, preferably at 32°C. In the simultaneous staining step, the sample may be kept at a first temperature, and then at a second temperature that is lower than the first temperature. In such a case, the period of keeping at the first temperature may be longer than, the same as, or shorter than the period of keeping at the second temperature. Preferably, the first temperature is 23 to 37°C, and the second temperature is 2 to 6°C.

The length of time of the exposure in the simultaneous staining step is not limited as long as the biological tissue-staining reagent penetrates into the inside of the sample. The sample is soaked in the biological tissue-staining reagent for, for example, 3 weeks, 2 weeks, 1 week, or 2 to 6 days.

FIG. 3 shows an example of the steps constituting a biological tissue-staining method in a case where nuclear staining and immunostaining are simultaneously carried out for a sample that is a mouse whole brain. In the biological tissue-staining method shown in FIG. 3, the nuclear staining step and the immunostaining step are simultaneously carried out. Therefore the period required for the nuclear staining and the immunostaining can be shortened compared to a case where the nuclear staining and the immunostaining are not simultaneously carried out. Further, the enzyme reaction step for improvement of penetrability of the antibody, shown in FIG. 2A, is not required in the biological tissue-staining method shown in FIG. 3.

Since, in the biological tissue-staining method according to the present embodiment, staining with a dye and immunostaining with an antibody are simultaneously carried out for the sample, the time required for staining the sample can be largely reduced as shown below in Example 18.

Examples of the configuration of a biological tissue-staining kit suitable for the biological tissue-staining method according to the present embodiment include the following.

### [Biological Tissue-Staining Kit]

### (Configuration)

1. Staining buffer (1 × or 2× concentration)
2. Additives

### (Specification 1)

Staining buffer (1× or 2× concentration): (1×) 10 mM HEPES (pH 7.5), 10% Triton X-100, 500 mM NaCl, 0.5% (or 1%) casein
Additives: (2×) 5% nicotinic acid hydrazide, 10% pyrazine

### (Specification 2)

Staining buffer (1× or 2× concentration): (1×) 10 mM HEPES (pH 7.5), 10% Triton X-100, 500 mM NaCl, 0.5% (or 1%) casein
Additives: (2×) 5% pyrazine, 10% N,N-diethylnicotinamide

### (Specification 3)

Staining buffer (1 × or 2× concentration): (1 ×) 10 mM HEPES (pH 7.5), 10% Triton X-100, 500 mM NaCl, 0.5% (or 1%) casein
Additives: (2×) 5% nicotinic acid hydrazide, 10% pyrazine, (2×) 2 to 3% 2-[2-(dimethylamino)ethoxy] ethanol

### (Specification 4)

Staining buffer (1 × or 2× concentration): (1×) 10 mM HEPES (pH 7.5), 10% Triton X-100, 500 mM NaCl, 0.5% (or 1%) casein
Additives: (2×) 5% pyrazine, 10% N,N-diethylnicotinamide, (2×) 2 to 3% 2-[2-(dimethylamino)ethoxy] ethanol

### (Specification 5)

Staining buffer (1 × or 2x concentration): (1 ×) 10 mM HEPES (pH 7.5), 10% Triton X-100, 500 mM NaCl, 0.5% (or 1%) casein
Additive: (2×) 2 to 3% 2-[2-(dimethylamino)ethoxy]ethanol

### Embodiment 3

The initial concentrations of the antibody and the dye in the biological tissue-staining reagent during the exposure of the sample to the biological tissue-staining reagent are very important for allowing the antibody and the dye to penetrate into the sample three-dimensionally. For inexpensively securing high initial concentrations of the antibody and the dye, the antibody or the dye needs to be included in a very small amount of the biological tissue-staining reagent. However, in cases where the amount of the biological tissue-staining reagent is small, soaking of the whole sample in the reagent is impossible. Thus, in the biological tissue-staining method according to the present embodiment, a phase separation-inducing reagent for phase separation from water is used in the staining step of exposing the sample to the biological tissue-staining reagent. A biological tissue-staining method according to the present embodiment is described below mainly regarding the difference from Embodiment 1.

The phase separation-inducing reagent may be any of a liquid phase, gas phase, and solid phase as long as the reagent enables phase separation from water, which is the main component of the biological tissue-staining reagent. The phase separation-inducing reagent is, for example, an oil immiscible with water, such as mineral oil. Preferably, the phase separation-inducing reagent is hardly immiscible with the surfactant contained in the biological tissue-staining reagent, has a specific gravity close to the specific gravity of the biological tissue-staining reagent, and does not have an excessive viscosity. Examples of reagents satisfying these conditions include KF-96 (manufactured by Shin-Etsu Silicone Co., Ltd.; viscosity, 50), which is a dimethyl silicone oil.

In the staining step in the biological tissue-staining method according to the present embodiment, a biological tissue-staining reagent according to the above embodiment including at least one of an antibody or a dye is mixed with a sample and a phase separation-inducing reagent. Since the phase separation-inducing reagent causes phase separation from water, the sample and the biological tissue-staining reagent are covered by the phase separation-inducing reagent from the outside. The sample is exposed to the biological tissue-staining reagent separated from the phase separation-inducing reagent, and at least one of the antibody or the dye penetrates into the sample. By the combined use of the phase separation-inducing reagent, the dye or the antibody in the biological tissue-staining reagent can be concentrated compared to the case where the phase separation-inducing reagent is not used, even with the same amounts of the antibody and the dye.

The concentration of the dye in the biological tissue-staining reagent according to the present embodiment is, for example, 10 to 100 µg/mL, 20 to 80 µg/mL, or 30 to 50 µg/mL. The concentration of the antibody in the biological tissue-staining reagent according to the present embodiment is, for example, 0.5 to 500 µg/mL, preferably 30 to 400 µg/mL, especially preferably 50 to 200 µg/mL.

The biological tissue-staining method according to the present embodiment using the phase separation-inducing reagent is effective for increasing the initial concentration of an antibody or a dye not only in cases where a sample is exposed to the biological tissue-staining reagent according to Embodiment 1, but also in cases where a sample is exposed to a conventional staining buffer. For example, in cases where PBS containing a nonionic surfactant is used as a staining buffer, at least one of an antibody or a dye may be included in the staining buffer, and the resulting buffer may be mixed with a sample and a phase separation-inducing reagent.

By the phase separation-inducing reagent in the biological tissue-staining method according to the present embodiment, the initial concentration of the antibody or the dye can be increased in the biological tissue-staining reagent to which the sample is exposed, compared to cases where the same amount of the antibody or the dye is used without using the phase separation-inducing reagent. Thus, the antibody or the dye can be allowed to penetrate into the sample inexpensively and efficiently (see Example 19 below).

In another embodiment, a biological tissue-staining kit including: a staining buffer; and the phase separation-inducing reagent; is provided. The staining buffer in the biological tissue-staining kit is preferably the biological tissue-staining reagent according to Embodiment 1. In another embodiment, a biological tissue-staining auxiliary material including a substance that causes phase separation from water is provided.

The phase separation-inducing reagent according to the present embodiment may also be applied to the simultaneous staining step in Embodiment 2. The time required for staining of the sample can thus be further largely shortened (see Example 20 below).

Regarding the configuration of a biological tissue-staining kit suitable for the biological tissue-staining method according to the present embodiment, for example, a phase separation-inducing reagent is included in the biological tissue-staining kit according to Embodiment 1. Examples of the specification of the phase separation-inducing reagent include mineral oil or dimethyl silicone oil (KF-96 (viscosity, 50), manufactured by Shin-Etsu Silicone Co., Ltd.).

### Examples

The present disclosure is described more concretely by way of the following Examples. However, the present disclosure is not limited by Examples.

The compositions of the buffers for defatting treatment and dye staining, and the immunostaining buffer, used in the Examples below are as follows.
CUBIC-L:
   10% by weight N-butyldiethanolamine (manufactured by Tokyo Chemical Industry Co., Ltd.; #B0725)
   10% by weight Triton X-100 (manufactured by Nacalai Tesque, Inc.; #12967-45)
   Double-distilled water
TS:
   10% by weight Triton X-100
   500 mM NaCl
   0.05% by weight NaN₃
CUBIC-1A:
   10% by weight Triton X-100
   5% by weight Quadrol (manufactured by Tokyo Chemical Industry Co., Ltd.; #T0781)
   10% by weight urea (manufactured by Nacalai Tesque, Inc.; #35904-45)
   25 mM NaCl (manufactured by Nacalai Tesque, Inc.; #31319-45)
   Double-distilled water
H-S:
   10 mM HEPES (pH 7.5) (manufactured by Nacalai Tesque, Inc.; #17514-15)
Dye Staining Buffer A:
   10% by weight Triton X-100
   500 mM NaCl
   5% by weight Quadrol
   Double-distilled water
Dye Staining Buffer B:
   10% by weight Triton X-100
   500 mM NaCl
   5% by weight Quadrol
   10% by weight urea
   Double-distilled water
Immunostaining Buffer A:
   10 mM HEPES (pH 7.5)
   10% by weight Triton X-100
   200 mM NaCl
   0.05% by weight NaN₃
Immunostaining Buffer B:
   10 mM HEPES (pH 7.5)
   0.1% by weight or 5% by weight Triton X-100
   200 mM Arginine-HCl
   0.5% (w/v) Casein (manufactured by Wako Pure Chemical Industries, Ltd.; #030-01505)
   0.05% by weight NaN₃
Immunostaining Buffer C:
   10 mM HEPES (pH 7.5)
   5% by weight or 10% by weight Triton X-100
   200 mM NaCl
   0.5% (w/v) Casein
   0.05% by weight NaN₃
Immunostaining Buffer D:
   10 mM HEPES (pH 7.5)
   10% by weight Triton X-100
   200 mM NaCl
   0.5 (w/v) Casein
   2.5% by weight Quadrol
   0.05% by weight NaN₃
Immunostaining Buffer E:
   10 mM HEPES (pH 7.5)
   10% by weight Triton X-100
   200 mM NaCl
   0.5 (w/v) Casein
   0.05% by weight NaN₃
Staining Buffer F:
   10 mM HEPES (pH 7.5)
   10% by weight Triton X-100
   500 mM NaCl
   0.5% (w/v) Casein
   0.05% by weight NaN₃

### Example 1: 3D Nuclear Staining with High-Concentration Salt and Surfactant

### (Sample Preparation)

A 5-month-old ICR mouse (manufactured by CLEA Japan, Inc.) was euthanized with an excessive amount (>100 mg/kg; intraperitoneal) of pentobarbital (pentobarbital sodium salt (manufactured by Nacalai Tesque, Inc.; #02095-04)), and perfusion fixation was transcardially carried out with 10 mL of phosphate-buffered saline (PBS) containing about 10 U/mL heparin, and then with 20 to 30 mL of PBS containing 4% PFA. Subsequently, the whole brain was removed from the head, and the whole brain was further fixed at 4°C for 8 to 24 hours in PBS containing 4% PFA. The fixed whole brain was soaked in CUBIC-L at 37°C for 5 days, to obtain a defatted whole brain. A defatted cerebral hemisphere cut out from the defatted whole brain was provided as a sample.

### (Nuclear Staining)

The sample was soaked in TS containing 5 µg/mL PI (manufactured by Molecular Probes; #P21493), and staining was carried out at 37°C for 2 days. At positions 2, 3, and 4 mm distant from the outside of the stained sample, frozen sections having a thickness of 50 µm were prepared.

### (Image Acquisition and Image Processing)

The frozen sections were observed under a fluorescence microscope (BX51, manufactured by Olympus Corporation) equipped with an objective lens (UPlanSApo 4×, N. A. = 0.16), a filter, a dichroic mirror compatible with the filter, and a sCMOS camera (ORCA-Flash4.0, manufactured by Hamamatsu Photonics K. K.). Using an electric x-y stage (manufactured by PRIOR) and software (cellSens Dimension 1.18, manufactured by Olympus Corporation), 16-bit images covering the respective whole frozen sections were obtained. Fiji/ImageJ was used for computer processing of the images.

### (Results)

FIG. 4 shows nuclear staining images of the frozen sections at positions 2, 3, and 4 mm distant from the outside. Uniform nuclear staining images could be obtained throughout the whole frozen sections.

### Example 2: 3D Nuclear Staining at Different Salt Concentrations

### (Sample Preparation)

The whole brain of an 8-week-old C57BL/6 mouse (Japan SLC, Inc.) fixed in the same manner as in Example 1 was soaked in CUBIC-1A at 37°C for about 10 days, to obtain a defatted whole brain. A defatted cerebral hemisphere cut out from the whole brain was provided as a sample.

### (Nuclear Staining)

The sample was soaked in H-S containing 2 µg/mL PI or SYTO 16 (1:150; manufactured by Thermo Fisher Scientific; #S7578), or in CUBIC-1A mixed with 2 µg/mL PI or SYTO 16 (1:150), and staining was carried out at 32°C for 24 hours. For investigating the effect of the NaCl concentration, the NaCl concentration in CUBIC-1A was set to 25 mM or 500 mM, and NaCl was added to H-S to 5 mM or 500 mM. The sample was washed with PBS, and then soaked in PBS containing 40% by weight sucrose. From each sample obtained, a frozen section was prepared. For post-2D staining, the frozen section was soaked in PBS containing DAPI (1:500; manufactured by Dojindo Molecular Technologies; #D0523) at room temperature for 30 minutes. The obtained frozen section was observed under a fluorescence microscope in the same manner as in Example 1.

### (Results)

FIG. 5A and FIG. 5B show nuclear staining images of the frozen sections of the samples 3D-stained with PI and SYTO 16, respectively. With either PI, which is an ionizing dye, or SYTO 16, which is an ionizing lipophilic dye, more uniform nuclear staining images could be obtained throughout the whole frozen sections under the conditions of H-S and CUBIC-1A at the higher NaCl concentration. The post-2D staining with DAPI was also found to be capable of detecting nuclei.

### Example 3: 3D Nuclear Staining with Various Dyes

### (Sample Preparation)

A whole brain fixed in the same manner as in Example 2 was soaked in CUBIC-1A at 37°C for about 10 days to carry out defatting treatment, and a cerebellar hemisphere was cut out to obtain a sample.

### (Nuclear Staining)

The sample was soaked in CUBIC-1A whose NaCl concentration was set to 500 mM, and staining was carried out at 32°C for 2 days. The following dyes were used: DAPI (1:400), SYTOX-G (1:2500; manufactured by Thermo Fisher Scientific; #S7020), RedDot2 (1:150; manufactured by Biotium; #40061), or NeuroTrace (1:150; manufactured by Thermo Fisher Scientific; #N21483). Thereafter, from each sample, a frozen section was prepared in the same manner as in Example 2. The obtained frozen section was observed under a fluorescence microscope in the same manner as in Example 1.

### (Results)

FIG. 6 shows nuclear staining images of the frozen sections of the samples stained with DAPI, SYTOX-G, RedDot2, and NeuroTrace. With any of the dyes, a uniform nuclear staining image could be obtained throughout the whole frozen section. Thus, CUBIC-1A with an increased salt concentration was found to be capable of allowing uniform 3D nuclear staining with various dyes.

### Example 4: 3D Nuclear Staining with Dye Staining Buffers

### (Sample Preparation)

A defatted cerebral hemisphere sample was obtained in the same manner as in Example 1 except that an 8-week-old C57BL/6 mouse was used.

### (Nuclear Staining)

The sample was soaked in Dye Staining Buffer A mixed with SYTOX-G (1:2500), and staining was carried out at 37°C for 3 days. On the other hand, the sample was similarly stained with Dye Staining Buffer B, which was prepared by further including urea to Dye Staining Buffer A. From each sample, a frozen section was prepared in the same manner as in Example 1, and the frozen section was observed.

### (Results)

FIG. 7A and FIG. 7B show nuclear staining images of the frozen sections of the samples stained with Dye Staining Buffer A and Dye Staining Buffer B, respectively. Uniform nuclear staining images could be obtained throughout the whole frozen sections irrespective of whether urea was present or not. As a whole, Dye Staining Buffer A, which does not contain urea, produced a stronger signal than Dye Staining Buffer B, which contains urea.

### Example 5: 3D Immunostaining with Moderate Concentration of Salt and Surfactant

### (Sample Preparation)

From a cerebral hemisphere defatted in the same manner as in Example 1, a sample with a thickness of about 3 mm was obtained by coronal section.

### (Immunostaining)

A mouse anti-NeuN antibody labeled with the fluorescent dye Alexa 488 (Anti-NeuN-A488 antibody, manufactured by Merck Millipore; MAB377X) was mixed with Immunostaining Buffer A at a concentration of 10 µg/mL, and the sample was soaked therein, followed by carrying out staining at 32°C for 4 days. NeuN is a marker for neuronal nuclei. A frozen section with a thickness of 50 µm was prepared by coronal section from the stained sample, and the frozen section was observed in the same manner as in Example 1.

### (Results)

FIG. 8 shows an immunostaining image of the frozen section. A uniform staining image of neuronal nuclei could be obtained throughout the whole frozen section.

### Example 6: Immunostaining with Different Concentrations of Surfactant

### (Sample Preparation)

The whole brain of an 8-week-old C57BL/6 mouse fixed in the same manner as in Example 1 was soaked in CUBIC-1A at 37°C for about 10 days, to obtain a defatted whole brain as a sample. From the sample, frozen sections of the whole brain with a thickness of 50 µm were prepared by sagittal section, and the sections were used for staining.

### (Immunostaining)

The frozen sections were soaked in Immunostaining Buffer B or Immunostaining Buffer C mixed with 1 µg/mL anti-Sst antibody (manufactured by Merck Millipore; #MAB354) and Fab-anti-rat labeled with the fluorescent dye Alexa 594 (Fab-anti-rat-A594, manufactured by Jackson ImmunoResearch laboratories Inc.; #112-587-008) at a weight ratio of 1:0.7 to 1:3, and staining was carried out at 32°C overnight. The frozen sections after the staining were observed in the same manner as in Example 1.

### (Results)

FIG. 9A and FIG. 9B show immunostaining images of frozen sections stained with Immunostaining Buffer B. A stronger signal could be obtained at the higher Triton X-100 concentration. FIG. 10A and FIG. 10B show immunostaining images of frozen sections stained with Immunostaining Buffer C. A stronger signal could be obtained in the case where the Triton X-100 concentration was 10% by weight than in the case where the Triton X-100 concentration was 5% by weight.

### Example 7: Comparison between Immunostaining Buffer and PBST Buffer

### (Sample Preparation)

The whole brain of an 8-week-old C57BL/6 mouse fixed in the same manner as in Example 1 was soaked in CUBIC-L for 3 days, to obtain a defatted sample. From the sample, a frozen section of the whole brain with a thickness of 50 µm was prepared by sagittal section, and the section was used for staining.

### (Immunostaining)

The frozen section was soaked in Immunostaining Buffer E mixed with 1 µg/mL mouse anti-NeuN antibody and Fab-anti-mouse IgG₁-A594 at a weight ratio of about 1:0.75, and staining was carried out at 32°C for 24 hours. From the stained sample, a frozen section was prepared. For comparison, a frozen section was stained also with PBST buffer (0.1% (v/v) Triton X-100 and 3% donkey serum (manufactured by Sigma-Aldrich, #D9663)), and the stained frozen section was observed in the same manner as in Example 1.

### (Results)

FIG. 11 shows staining images of neuronal nuclei of the frozen section sample stained with Immunostaining Buffer E and the frozen section sample stained with PBST buffer. Based on the magnified images of the areas indicated with "1" and "2" in the top left image, Immunostaining Buffer E was shown to better improve the signal-noise ratio compared to PBST buffer.

### Example 8: Comparison with Other 3D Clearing-Staining Methods

For comparison among AbScale, which is an already reported 3D clearing-staining method (Hiroshi Hama and 10 other authors, "ScaleS: an optical clearing palette for biological imaging", Nat. Neurosci., 2015, 18, 1518-1529), iDISCO+ (version in December 2016; https://idisco.info/idisco-protocol/), which is disclosed in Non Patent Literature 2, and a staining method using Immunostaining Buffer E, a cerebral hemisphere of an 8-week-old C57BL/6 mouse fixed in the same manner as in Example 1 was defatted by soaking in CUBIC-L at 37°C for 3 days, and the degree of penetration of antibody into the defatted sample was compared.

In AbScale (original), the fixed sample was soaked sequentially in ScaleS0, ScaleA2, AcaleB4, and ScaleA2 in the order illustrated in FIG. 12, and then the sample was washed, followed by soaking the sample in 1.6 mL of AbScale solution (PBS containing 0.33 M urea and 0.5% (w/v) Triton X-100) mixed with a mouse anti-NeuN antibody labeled with Alexa 488 (5 µg/mL), and staining at 37°C. ScaleS0 is a solution of 20% (w/v) D-(-)-sorbitol (manufactured by Nacalai Tesque, Inc.; #32021-95), 5% (w/v) glycerol (manufactured by Nacalai Tesque, Inc.; #17018-25), 1 mM methyl-β-cyclodextrin (manufactured by Tokyo Chemical Industry Co., Ltd.; #M1356), 1 mM γ-cyclodextrin (manufactured by Wako Pure Chemical Industries, Ltd.; #037-10643), 1% (w/v) N-acetyl-L-hydroxyproline (manufactured by Santa Cruz Biotechnology, Inc.; #sc-237135), and 3% (v/v) dimethyl sulfoxide (manufactured by Nacalai Tesque, Inc.; #35624-15) in PBS. ScaleA2 is a solution of 10% (w/v) glycerol, 4M urea, and 0.1% (w/v) Triton X-100 in distilled water. ScaleB4 is a solution of 8M urea in distilled water.

In iDISCO+ (original), the fixed sample was dehydrated by sequential treatment with 20%, 40%, 60%, 80%, and 100% methanol in the order illustrated in FIG. 12, and shaken overnight in 66% dichloromethane (DCM) / 33% methanol. Subsequently, the sample was washed with 100% methanol, and treated overnight in methanol containing 5% hydrogen peroxide. Thereafter, the sample was subjected to sequential re-dehydration with 80%, 60%, 40%, and 20% methanol, and then washed. The sample was then treated with a permeabilization solution and a blocking solution.

In the staining with a primary antibody in iDISCO+ (original), the sample was stained at 37°C using 1.6 mL of a primary staining buffer (PBS containing 5% DMSO, 3% donkey serum, 0.2% (v/v) Tween-20, and 10 µg/mL heparin) mixed with mouse anti-NeuN (10 µg/mL). In the staining with a secondary antibody, the sample was stained at 37°C using a secondary staining buffer (PBS containing 3% donkey serum, 0.2% (v/v) Tween-20, and 10 µg/mL heparin) mixed with anti-mouse secondary IgG (A488) (10 µg/mL).

In the staining using Immunostaining Buffer E, the sample was soaked in 50% CUBIC-L and 100% CUBIC-L in the order illustrated in FIG. 12. After washing, the sample was soaked in Immunostaining Buffer E mixed with mouse monoclonal anti-NeuN IgG (10 µg/mL) and anti-mouse secondary Fab labeled with A488 (manufactured by Jackson ImmunoResearch laboratories Inc.; #115-547-185) at a weight ratio of 1:1, and staining was carried out at 32°C.

On the other hand, as shown in FIG. 12, the sample was stained under the same staining conditions as in AbScale (original) and iDISCO+ (original) except that Immunostaining Buffer E was used.

### (Results)

Based on FIG. 13, which shows images of the frozen sections of the samples, staining images of neuronal nuclei inside the samples could not be completely obtained in the cases of staining by AbScale (original) and iDISCO+ (original). In contrast, a staining image of neuronal nuclei inside the sample could be favorably obtained in the case of staining using Immunostaining Buffer E according to the present Example. In AbScale and iDISCO+, the staining images of neuronal nuclei inside the samples could be evidently improved by staining using Immunostaining Buffer E. Thus, Immunostaining Buffer E was found to be capable of allowing sufficient penetration of antibody to a biological tissue even in cases of combination with an existing 3D clearing-staining method. In the staining method using Immunostaining Buffer E according to the present Example, the penetration efficiency of the antibody to the sample could be improved by the control of the antibody concentration, the temperature during the use of the Fab antibody and the staining, and the like as well as by the use of Immunostaining Buffer E.

### Example 9: Two-Color 3D Staining of Whole Brain

A protocol (hereinafter referred to as "CUBIC-HV") according to the following Example, which is widely applicable to 3D tissue staining and image analysis, is illustrated in FIG. 2A. For nuclear staining in CUBIC-HV, the Dye Staining Buffer B was used. For immunostaining in CUBIC-HV, the Immunostaining Buffer E was used. As shown in FIG. 2B, enzyme treatment was arbitrarily added to CUBIC-HV.

### (Sample Preparation)

The whole brain of an 8-week-old C57BL/6 mouse fixed in the same manner as in Example 1 was soaked in CUBIC-L at 37°C for 3 days, to obtain a defatted sample.

### (Nuclear Staining)

The sample was washed with PBS, and stored in PBS containing 0.05% NaN₃ at 4°C until use. The sample was soaked in Dye Staining Buffer B mixed with SYTOX-G (1:2500), and staining was carried out at 37°C for 5 days.

### (Enzyme Treatment)

The sample was washed, and then subjected to enzyme treatment. In the enzyme treatment, the sample was treated at 37°C for 24 hours in CAPSO buffer (containing 10 mM CAPSO (manufactured by Sigma-Aldrich; #C2278) and 150 mMNaCl; pH 10) containing hyaluronidase (manufactured by Sigma-Aldrich; #H4272 or H3884; 3 mg/mL).

### (Immunostaining)

After the enzyme treatment, the sample was washed with PBS, and soaked in Immunostaining Buffer E that was mixed with 10 µg/mL mouse anti-NeuN antibody (manufactured by Merck Millipore; MAB377) and Fab-anti-mouse IgG₁ labeled with Alexa 594 (Fab-anti-mouse IgG₁-A594, manufactured by Jackson ImmunoResearch laboratories Inc.; #115-587-185; added such that the weight ratio to the mouse anti-NeuN antibody was about 1:0.5) and further supplemented with 2.5% by weight Quadrol. The sample was stained at 32°C for 7 days, and then at 4°C for 5 days. The sample after staining was cleared with CUBIC-R (RI adjustment). CUBIC-R is double-distilled water containing 45% by weight antipyrine and 30% by weight nicotinamide, buffered (pH, about 10) with 0.5% (v/w) N-butyldiethanolamine. The sample was washed with PBS, and postfixed in 1% formaldehyde (FA) at room temperature for 5 hours. For clearing, the sample was soaked in CUBIC-R diluted with water at a ratio of 1:1, at room temperature for 1 day. Subsequently, the sample was soaked in undiluted CUBIC-R at room temperature for 2 to 3 days. Finally, the sample was embedded in a gel prepared with 2% (w/v) agarose dissolved in CUBIC-R.

### (Image Acquisition and Image Processing)

3D observation of the sample was carried out using a light-sheet microscope (LSFM) equipped with left and right sheet illumination units (manufactured by Olympus Corporation) and two macro-zoom microscopes (MVX10, manufactured by Olympus Corporation) placed in the front side and the rear side of the sample. For switching between the left and right sheet illumination paths, a fiber-connected diode or DPSS laser (Omicron; SOLE-6 with λ=488, 532, 594, and 642 nm) was connected to the sheet illumination units through a collimator and a beam mirror. Sheet illumination was generated with a sheet illumination unit having a cylindrical lens. The thickness of the sheet illumination was adjusted within the range of about 5 to 10 µm using a mechanical slit. In the LSFM observation in the present Example, a 0.63× object lens (MVPLAPO 0.63X N. A. = 0.15, WD=87 mm; manufactured by Olympus Corporation), an MVX10 optical zoom (1.25×), 488 nm and 590 nm lasers, single-band path filters with a diameter of 32 mm (520/44 nm and 641/75 nm; manufactured by Semrock), a tube lens (MVX-TV XC, manufactured by Olympus Corporation), and a sCMOS camera (Zyla 5.5; manufactured by Andor) were used. For acquisition of sample images, a sample holder and a cleared sample embedded in a gel were connected to an electric x-y-z stage (x and y stages: MTS50/M-Z8E, manufactured by Thorlabs; z stage: M-112.1DG, manufactured by Physik instrumente), and placed in a sample chamber having illumination and imaging windows, filled with an RI adjustment oil mixture (RI, about 1.51) including HIVAC-F4 and mineral oil. The sample was scanned at a step size of 9 µm in the z direction, to obtain 16-bit images. A plurality of z-stack data was obtained by moving the focus of the sheet illumination such that the confocal parameter of the beam waist covers the entire sample in each x-y image after tiling. All electronic apparatuses were controlled by LabVIEW software (manufactured by National Instruments).

The brain image obtained using LSFM was processed as follows using Fiji/ImageJ. First, six images obtained by moving the focus of the sheet illumination (three locations × light illumination from left and right) at each z-position were tiled to construct one image. The x-position of the edge in the tiling was determined by collecting images while moving the focus position of the light sheet at the z-position at the center of the sample, and comparing the signal contrast of each image. Before the tiling, the mean intensity was equalized among the images obtained by moving the focus position of the light sheet in each z-step. After the tiling, the 16-bit image obtained was converted to an 8-bit image, and the blank region was eliminated. Subsequently, noise signals were removed by the following filters. 1) After selecting the noise pixels outside the brain region based on an intensity threshold, the "remove outlier" function of ImageJ is applied. 2) After selecting the noise pixels with an intensity threshold higher than the intensities of the staining signals, the "remove outlier" function of ImageJ is applied, or the intensity is replaced by 0. 3) After selecting noise pixels by combination of an intensity threshold and manual selection, the "clear" function of ImageJ is applied. When necessary, the x-y position was manually matched between different channels.

The z-stack data obtained using LSFM and processed as described above were reconstructed using Imaris software (version 8.4, manufactured by Bitplane), to visualize the data as a pseudocolor image. The brightness and the contrast of the pseudocolor image was adjusted.

### (Results)

FIG. 14A shows an image showing the signal of SYTOX-G and an image showing the signal of NeuN, and an image prepared by merging these images. Using LSFM, a z-stack image covering the sample with an almost isotropic voxel size (8.3 × 8.3 × 9 µm) was obtained. FIG. 14B shows sagittal-plane (y-z) and coronal-plane (x-z) images of the data shown in FIG. 14A. Uniform staining and images could be acquired at an almost isotropic, cellular-level resolution.

### Example 10: Multicolor 3D Staining of Whole Brain

Unlike genetic methods, tissue staining easily enables staining and visualization of a plurality of targets in one sample. In the present Example, a mouse whole brain was 3D-stained according to CUBIC-HV as follows with BOBO-1, and three different antibodies related to GABA neurons.

The whole brain of an 8-week-old C57BL/6 mouse fixed in the same manner as in Example 1 was soaked in CUBIC-L for 3 days, to obtain a defatted sample. The sample was washed with PBS, and stored in PBS containing 0.05% NaN₃ at 4°C until use. The sample was soaked in Dye Staining Buffer B mixed with BOBO-1 (1:400), and staining was carried out at 37°C for 5 days. The sample was washed, and then subjected to enzyme treatment with hyaluronidase in the same manner as in Example 9.

After the enzyme treatment, the sample was washed with PBS, and soaked in Immunostaining Buffer E mixed with mouse monoclonal anti-PV IgG₁/Fab-Cy3 (1:50; manufactured by Swant; #PV235), rat monoclonal anti-Sst IgG_{2b}/Fab-A594 (1:10; manufactured by Millipore, #MAB354), and mouse monoclonal anti-Gad67 IgG₂ₐ/Fab-A647 (1:75; manufactured by Millipore; #MAB5406). Staining was carried out at room temperature for 7 weeks, and then at 4°C for 5 days. The sample was washed with PBS, and then postfixed in 1% FA at room temperature for 5 hours. After washing the sample, RI adjustment was carried out with CUBIC-R, and a 3D image was obtained using LSFM as described above.

### (Results)

FIG. 15 shows images of the channels for PV (panel b), Sst (panel c), and Gad67 (panel d) as well as the channel for BOBO-1. The voxel size was 8.3 × 8.3 × 9 µm. Panel e of FIG. 15 shows a superimposed image of these four channels. Panel f of FIG. 15 and panel g of FIG. 15 show magnified images of part of the brain on a horizontal plane (x-y). Panel h of FIG. 15 shows a coronal-plane (x-z) image of a part shown in panel e of FIG. 15. Each of panel i of FIG. 15 and panel j of FIG. 15 shows a magnified image of an area shown in panel h of FIG. 15. The results demonstrated that identification of signals from different fluorescence channels is possible using appropriate types of antibodies (host species and isotypes), dyes, excitation lights, and band-path emission filters.

### Example 11: Multicolor 3D Staining of Whole Brain Expressing Fluorescent Protein

In the present Example, 3D staining of a whole brain labeled with a fluorescent protein was carried out using two kinds of antibodies according to CUBIC-HV.

For an 8-week-old Thy1-YFP-H transgenic mouse (Feng, G. P. and eight other authors, "Imaging neuronal subsets in transgenic mice expressing multiple spectral variants of GFP", Neuron, 2000, 28, 41-51), the whole brain was fixed in the same manner as in Example 1, and soaked in CUBIC-L for 3 days to obtain a defatted sample. The sample was washed, and then subjected to enzyme treatment with hyaluronidase in the same manner as in Example 9. The brain of a Thy1-YFP-H transgenic mouse expresses YFP.

After the enzyme treatment, the sample was washed with PBS, and soaked in Immunostaining Buffer E mixed with mouse monoclonal anti-Dat IgGi/Fab-A594 (1:100; manufactured by Abcam; #ab128848) and goat polyclonal anti-ChAT IgG/Fab-A647 (1:20; manufactured by Millipore; #AB144) and further supplemented with 2.5% by weight Quadrol. Staining was carried out at 32°C for 15 days, and then at 4°C for 5 days. The sample was washed with PBS, and then postfixed in 1% FA at room temperature for 5 hours. After washing the sample, RI adjustment was carried out with CUBIC-R+(M), and a 3D image was obtained using LSFM as described above. CUBIC-R+(M) is double-distilled water containing 45% by weight antipyrine and 30% by weight N-methylnicotinamide (manufactured by Tokyo Chemical Industry Co., Ltd.; #M0374), buffered (pH, about 10) with 0.5% (v/w) N-butyldiethanolamine.

### (Results)

Panel k of FIG. 16 shows a superimposed image of the channels for ChAT, Dat, and YFP. The voxel size was 8.3 × 8.3 × 9 µm. Panel 1 of FIG. 16 shows a magnified image of the area indicated by "l" in panel k of FIG. 16. Panel m of FIG. 16 shows an image of part of the brain on a horizontal plane. Panel n of FIG. 16 shows a magnified image of an area shown in panel m of FIG. 16. The image of the corticospinal tract, labeled with YFP, and the image of the projection pathway of dopaminergic neurons, immunostained with Dat, can be seen next to each other. Panels o to q of FIG. 16 are reconstructed sagittal-plane images for the area indicated by "o-q" in panel k of FIG. 16. The "*" symbols in Panels k to q of FIG. 16 indicate non-specific signals from blood vessels caused by insufficient perfusion of the sample.

As shown in FIG. 16, the YFP signal was clearly observed with stronger intensity together with the signals of immunostained Dat and ChAT. Thus, CUBIC-HV using the Immunostaining Buffer E according to the present Example was shown to be capable of generating an image in which the respective targets are labeled with different dyes, and in which localization of those respective targets can be distinguished, without reducing the FP signal expressed in brain.

### Example 12: Selection of Antibodies for 3D Staining

Using a half brain of an 8-week-old C57BL/6 mouse defatted with CUBIC-L or CUBIC-1A in the same manner as in Example 1 or Example 2, various antibodies were stained according to the CUBIC-HV described above. In image analysis, the signal intensity, SBR, compatibility with enzyme treatment, and penetration efficiency were studied to identify antibodies preferred for 3D staining by CUBIC-HV (see Table 1 and Table 2).

In the enzyme treatment, the sample was subjected to the hyaluronidase treatment, and in addition, for some antibodies, to treatment with carbonate buffer (containing 50 mM calcium carbonate (manufactured by Nacalai Tesque, Inc.; #31310-35) and 50 mM sodium hydrogen carbonate (manufactured by Nacalai Tesque, Inc.; #31213-15)) that contains collagenase-P (manufactured by Sigma-Aldrich; #11213857001; 1 mg/mL) and whose pH was adjusted to 10 by addition of 150 mM NaCl and 25 to 100 µM EDTA, at 37°C for 18 to 24 hours. Further, to the Immunostaining Buffer E, Quadrol and urea were added as appropriate for improving the signal intensity, SBR, and penetration efficiency.

**[Table 1]**

| No. | Target Protein | Host | Clonality | Target cell type/ structure | Supplier | Catalog Number | Concentration | Dilution | Buffer used for defatting treatment | Temperature during 3D staining | Compatibility with enzyme treatment (option)' | Additive" |
|---|---|---|---|---|---|---|---|---|---|---|---|---|
| 1 | NeuN | Mouse | Monoclonal IgG1 | Neuron nuclei | Millipore | MAB377 (Lot# 2592741) | 1 mg/mL | 1/100 | CUBIC-L | 32°C | H: (+), C: (+) | Quadrol |
| 2 | NeuN | Rabbit | Polyclonal | Neuron nuclei | Millipore | ABN78 (Lot# 2885346) | 1 mg/mL | 1/100 | CUBIC-L | 32°C | H: (+), C: (+) | Quadrol |
| 3 | Calbindin D28K | Rabbit | Polyclonal | Inhibitory neuron | Sigma | C2724 (Lot# 093M4801) | 0.68 mg/mL | 1/50 | CUBIC-L | 32°C | H: (+), C: (+) | Quadrol / 0.5 M Urea |
| 4 | Parvalbumin (PV) | Mouse | Monoclonal IgG1 | Inhibitory neuron | Swant | PV235 (Lot# 10-11(F)) | serum | 1/50 | CUBIC-L | Room temperature | H: (+), C: (+) | None |
| 5 | Somatostatin (Sst) | Rat | Monoclonal IgG2b | Inhibitory neuron | Millipore | MAB354, clone YC7 (Lot# 3005269) | 0.1 mg/mL | 1/10 | CUBIC-L | Room temperature | H: (+), C: (+) | Quadrol |
| 6 | Glutamic Acid Decarboxylase (Gad) 67 specific | Mouse | Monoclonal IgG2a | Inhibitory neuron | Millipore | MAB5406, clone 1G10.2 (Lot# 2844575) | 1 mg/mL | 1/100 | CUBIC-L | 32°C | H: (+), C: (+) | Quadrol |
| 7 | Glutamic Acid Decarboxylase (Gad) 65/67 | Mouse | Monoclonal IgG1 | Inhibitory neuron | MBL | M018-3 (Lot# 024) | 1 mg/mL | 1/50 | CUBIC-L | 32°C | H: (+), C: (+) | Quadrol / 1 M Urea |
| 8 | Tyrosine hydroxylase (Th) | Mouse | Monoclonal IgG2a | Dopamine neuron | Santa Cruz | sc-25269 (Lot# H2510) | 0.2 mg/mL | 1/20 | CUBIC-L | 37°C | H: (+), C: (+) | Quadrol / 0.5 M Urea |
| 9 | Dopamine Transporter (Dat) | Mouse | Monoclonal IgG1 | Dopamine neuron | abcam | ab128848 (Lot# GR249640-3) | 1 mg/mL | 1/100 | CUBIC-L | 32°C | H: (+), C: (+) | Quadrol |
| 10 | Dopamine beta Hydroxylase (Dbh) | Rabbit | Monoclonal | Noradrenaline neuron | abcam | ab209487 (Lot# GR310529-2) | 0.53 mg/mL | 1/50 | CUBIC-L | Room temperature | H: (+), C: (+) | None |
| 11 | Choline Acetyltransferase (ChAT) | Goat | Polyclonal | Acetylcholine neuron | Millipore | AB144 (Lot# 3018862) | unknown | 1/20 | CUBIC-L | 32°C | H: (+), C: (+) | Quadrol |
| 12 | Choline Acetyltransferase (ChAT) | Rabbit | Monoclonal | Acetylcholine neuron | abcam | ab178850 (Lot# GR315911-3) | 2.1 mg/mL | 11200 | CUBIC-L | Room temperature | H: (+), C: (+) | Quadrol |
| 13 | Tryptophan hydroxylase 2 (Tph2) | Mouse | Monoclonal IgG7 | Serotonin neuron | Sigma | AMAb91108 (Lot# 02981) | 1 mg/mL | 1/100 | CUBIC-L | 32°C | H: (+), C: (+) | None |
| 14 | Copeptin | Goat | Polyclonal | AVP neuron | Santa Cruz | sc-7812 (Lot# J0604) | 0.2 mg/mL | 1/20 | CUBIC-L | 32°C | H: (+), C: (+) | Quadrol |
| 15 | LRPAP-1 | Rabbit | Polyclonal | Neuron subtypes | Sigma | HPA008001 (Lot# R02480) | 0.05 mg/mL | 1/10 | CUBIC-L | 32°C | H: (+), C: (+) | Quadrol |

| | | | | | | | | | | | | |
|---|---|---|---|---|---|---|---|---|---|---|---|---|
| *H, Hyaluronidase; C, Collagenase-P | | | | | | | | | | | | |

**2.5% Quadrol unless otherwise specified

**[Table 2]**

| No. | Target Protein | Host | Clonality | Target cell type/ structure | Supplier | Catalog Number | Concentratior Dilution | Dilution | Buffer used for defatting treatment | Temperature during 3D staining | Compatibility with enzyme treatment (option)' | Additive* |
|---|---|---|---|---|---|---|---|---|---|---|---|---|
| 16 | PKCa/PRKCA1 | Mouse | Monoclonal IgG2a | Neuron subtypes | Novus | NB600-201 (Lot# A-2) | 1 mg/mL | 1/75 | CUBIC-L | 32°C | H: (+), C: (+) | 1% Quadrol |
| 17 | Ionized calcium binding adapter molecule 1 (Iba1) | Rabbit | Polyclonal | Microglia | Wako | 019-19741 (Lot# CTF4377) | 0.5 mg/mL | 1/50 | CUBIC-L | Room temperature | H: (+), C: (+) | None |
| 18 | Glial Fibrillary Acidic Protein (GFAP) | Mouse | Monoclonal IgG1 | Astrocytes | Sigma | C9205 (Cy3 conj) (Lot# 022M4752V) | 1 mg/mL | 1/100 | CUBIC-L | 32°C | H: (+), C: (+) | None |
| 19 | Glial Fibrillary Acidic Protein (GFAP) | Mouse | Monoclonal IgG1 | Astrocytes | MBL | D097-3 (Lot# 012) | 1 mg/mL | 1/100 | CUBIC-L | 32°C | H: (+), C: (+) | Quadrol |
| 20 | Oligodendrocyte transcription factor 2 (Olig2) | Rabbit | Polyclonal | Oligodendrocytes | IBL | 18953 (Lot# 1D-104) | 0.1 mg/mL | 1/10 | CUBIC-1A | Room temperature | H: (+), C: (+) | None |
| 21 | Neurofilament (pan) | Mouse | Monoclonal IgG1 | Axon | invitrogen | 18-0171Z (Lot# 1634056A) | 0.05 mg/mL | 1/10 | CUBIC-L | 32°C | H: (+), C: (+) | Quadrol / 0.5 M Urea |
| 22 | Phospho-Neurofilament | Mouse | Monoclonal IgG1 | Axon | Biolegend | 801601, SMI31 (Lot# B222936) | 1 mg/mL | 1/50 | CUBIC-L | 32°C | H: (+), C: (+) | Quadrol / 2 M Urea |
| 23 | Microtubule-Associated Protein (MAP) 2 | Mouse | Monoclonal IgG1 | Neurite, dendrite | Abeam | ab11267 (Lot# GR319463-3) | 2.3 mg/mL | 1/200 | CUBIC-L | 32°C | H: (-), C: (+) | Quadrol |
| 24 | Synaptophysin | Mouse | Monoclonal IgGt | Pre-synapse | MBL | D073-3 (Lot# 013) | 1 mg/mL | 1/50 | CUBIC-L | 32°C | H: (+), C: (+) | 2.5-5% Quadrol |
| 25 | α-Smooth Muscle Actin (SMA) | Mouse | Monoclonal IgG2a | arterial smooth muscle | Sigma | A5228 (Lot# 074M4814V) | 2.2 mg/mL | 1/220 | CUBIC-L | 32°C | H: (+), C: (+) | Quadrol |
| 26 | c-Fos | Rabbit | Polyclonal | Activated neuron | Sigma | HPA018531 (Lot# E105812) | 0.2 mg/mL | 1/40 | CUBIC-1A | 37°C | H: (+), C: (+) | Quadrol |
| 27 | c-Fos | Mouse | Monoclonal IgG1 | Activated neuron | Abeam | clone 2H2 / ab208942(Lot# GR3187875-2) | 1 mg/mL | 1/150 | CUBIC-L | 32°C | H: (+), C: (+) | Quadrol |
| 28 | c-Fos | Rabbit | Monoclonal | Activated neuron | CST | 22505 (Lot# 9) | 0.26 mg/mL | 1/75 | CUBIC-L | 32°C | H: (-), C: (+) | None |
| 29 | Arc | Rabbit | Polyclonal | Activated neuron | Synaptic Systems | 156 003 (Lot# 156003/1-63) | 1 mg/mL | 1/100 | CUBIC-L | 32°C | H: (+), C: (+) | Quadrol |

| | | | | | | | | | | | | |
|---|---|---|---|---|---|---|---|---|---|---|---|---|
| *H,Hyaluronidase; C, Collagenase-P **2.5% Quadrol unless otherwise specified | | | | | | | | | | | | |

### Example 13: Formation of Reversible Antibody-Tissue Complex Due to Electrical Interaction

In CUBIC-HV, for secure labeling of a target in a tissue, application of pretreatment is expected for concentration of, and for improvement of penetrability of, an antibody to be used for immunostaining in the tissue. When an anionically charged polymer such as polyglutamic acid is incubated with an antibody under slightly acidic conditions, a shift toward the acidic side occurs relative to the isoelectric point, so that the cationically charged antibody forms a reversible complex with the polymer due to electrical interaction. In the present Example, formation of a complex between an antibody and a tissue due to electrical interaction was found.

A cerebral hemisphere of an 8-week-old C57BL/6 mouse fixed in the same manner as in Example 1 was soaked in CUBIC-L at 37°C for 3 days, to obtain a defatted sample. An anti-mouse IgG antibody labeled with Alexa 488 was mixed with 10 mM citrate buffer (pH 5) containing a predetermined concentration of NaCl, and the sample was incubated in the mixed liquid at 37°C overnight. In order to avoid generation of signals through antigen-antibody reaction in tissue staining, an antibody that does not cause antigen-antibody reaction with the tissue in principle was used. A blue-light illuminator was used for irradiation of the sample, and an image was captured with a digital camera equipped with an orange filter. Channels for the respective colors were separated using Fiji/ImageJ.

### (Results)

As shown in FIG. 17, a green signal was found on the tissue surface. Thus, the slightly acidic buffer was shown to allow formation of an antibody-tissue complex independent of antigen-antibody reaction.

### Example 14: Construction of Evaluation System for Antibody Penetration Efficiency

### (Sample Preparation)

From a pig brain slice fixed with 4% PFA for about 5 days, a small column of the cerebral cortex area was prepared using a biopsy punch with a diameter of 1.5 mm, to provide a sample. The sample was soaked in CUBIC-L at 37°C for about 2 days, to obtain a defatted sample to be used for staining.

### (Staining)

After the defatting treatment, the sample was washed with PBS, and soaked in Immunostaining Buffer E that was mixed with a mouse monoclonal anti-synaptophysin antibody (manufactured by Medical & Biological Laboratories Co., Ltd.; #D073-3; 20 µg/mL) and Fab-anti-mouse IgG₁ labeled with Alexa 594 (added such that the weight ratio to the anti-synaptophysin antibody was about 1:1) and further supplemented with 2.5% by weight Quadrol or the same amount of distilled water. The sample was stained at 32°C for 1 day. After the staining, the sample was washed with PB-Triton and PB, and a frozen section in the central portion of the sample was prepared. The frozen section after the staining was observed in the same manner as in Example 1.

### (Results)

FIG. 18A and B show images of the frozen sections of the samples stained with Immunostaining Buffer E to which distilled water or Quadrol, respectively, was added. Compared to FIG. 18A, wherein Quadrol was not added, FIG. 18B showed an increase in the staining performance inside the sample due to the addition of Quadrol, indicating that the penetration of the antibody was effectively increased. For quantification, a representative area showing almost uniform staining of the left and right sides of the column was selected using a micrographic image, and a brightness-value graph was prepared. In the graph, the ratio (peak-bottom ratio) of the maximum brightness (peak) in the left and right to the minimum brightness (bottom) in the sample was high in the sample to which Quadrol was not added. The ratio was low in the sample to which Quadrol was added, because of the increase in the staining performance inside the sample. Thus, using the peak-bottom ratio as a quantitative index of penetrability of an antibody into a tissue, a search was carried out for compounds as follows.

### Example 15: Search for Compounds That Increase Penetration Efficiency of Antibody into Tissue

After adding each of 426 kinds of listed compounds to Immunostaining Buffer E to 2.5% by weight, staining was carried out in the same manner as in Example 14. In addition, staining was carried out with each of 137 separately listed compounds in the same manner except that an anti-neurofilament antibody (anti-phospho-neurofilament SMI31, manufactured by Biolegend; #801601; 20 µg/mL) was used as the primary antibody instead of the anti-synaptophysin antibody. From micrographs of frozen sections, the degrees of antibody penetration at Hour 24 were evaluated based on the peak-bottom ratio.

### (Results)

The peak-bottom ratio of each compound in the case using the anti-synaptophysin antibody is shown in FIG. 19. In FIG. 19, the peak-bottom ratios in the case where water was added as a compound to Immunostaining Buffer E are indicated with arrows. The compounds that showed peak-bottom ratios lower than the peak-bottom ratio of Quadrol, which was about 2.8, are shown in Table 3 to Table 7. The compounds that showed peak-bottom ratios lower than the peak-bottom ratio of Quadrol, and that also showed evident penetration-increasing effect in the stained image, in the case using the anti-neurofilament antibody are shown in Table 8. Most of these compounds are compounds containing a cyclic amine, cyclic amide, chain amine, chain amide, sulfo group, or a combination thereof. From the compounds identified, compounds that do not strongly inhibit antibody staining, and compounds with which penetration-increasing effect can be obtained also for other plurality of antibodies, were especially selected, and used as additives in the following Examples.

**[Table 3]**

| ID | Compound name | CAS No. |
|---|---|---|
| #1363 | Pyridazine | 289-80-5 |
| #1568 | 2-Cyanoacetamide | 107-91-5 |
| #1291 | 5-Methyl-2-pyrrolidone | 108-27-0 |
| #1276 | Methacrylamide | 79-39-0 |
| #0855 | Nicotinamide | 98-92-0 |
| #1560 | N,N-Bis(2-cyanoethyl)formamide | 3445-84-9 |
| #0854 | Nicotinic Acid Hydrazide | 553-53-7 |
| #1571 | 4-Cyanopyridine | 100-48-1 |
| #0460 | Butyramide | 541-35-5 |
| #1145 | 4-Methylpyridine N-Oxide | 1003-67-4 |
| #1002 | Isonicotinamide | 1453-82-3 |
| #1121 | 1,5-Pentamethylenetetrazole | 54-95-5 |
| #0673 | 2,5-Dimethylpyrazine | 123-32-0 |
| #1376 | Thiomorpholine | 123-90-0 |
| #1070 | 2-Pyrrolidone | 616-45-5 |
| #1144 | 2-Pyridinecarboxylic Acid Hydrazide | 1452-63-7 |
| #0493 | 4-(2-Aminoethyl)morpholine | 2038-03-1 |
| #0444 | o-Sulfobenzimide Sodium Dihydrate | 128-44-9 |
| #0327 | 4-Acryloylmorpholine | 5117-12-4 |
| #0564 | epsilon-Caprolactam | 105-60-2 |
| #0766 | Methyl Carbamate | 598-55-0 |
| #1546 | Glutaronitrile | 544-13-8 |
| #0048 | 4-Acetamidocyclohexanol (cis- and trans- mixture) | 23363-88-4 |
| #0146 | 2-[2-(Dimethylamino)ethoxy]ethanol | 1704-62-7 |
| #0428 | 1,3- Bis[tris(hydroxymethyl) methylamino]propane | 64431-96-5 |
| #1141 | 2-Picolinamide | 1452-77-3 |
| #0582 | Sodium 4-Chlorobenzenesulfonate | 5138-90-9 |
| #0804 | Sodium Dehydrocholate | 145-41-5 |
| #0718 | Sodium 4-Chlorobenzenesulfinate | 14752-66-0 |
| #0608 | N,N-Diethylpropionamide | 1114-51-8 |

**[Table 4]**

| ID | Compound name | CAS No. |
|---|---|---|
| #1091 | 2-Piperidone | 675-20-7 |
| #1445 | 2,2,2-Trifluoroethanol | 75-89-8 |
| #0609 | N,N-Diethylnicotinamide | 59-26-7 |
| #0637 | 1,3-Dimethylthiourea | 534-13-4 |
| #1283 | N-Methylnicotinamide | 114-33-0 |
| #1574 | 3-Dimethylaminopropionitrile | 1738-25-6 |
| #0603 | N,N-Dimethylmethacrylamide | 6976-91-6 |
| #1431 | Tetrahydrothiophene 1,1-Dioxide | 126-33-0 |
| #1599 | Nicotine | 54-11-5 |
| #0805 | Sodium 2,5-Dichlorosulfanilate | 41295-98-1 |
| #1518 | Benzyl Benzoate | 120-51-4 |
| #0833 | Sodium 2,4-Dimethylbenzenesulfonate Monohydrate | 827-21-4 |
| #0355 | Acetoxime | 127-06-0 |
| #0795 | Diacetone Acrylamide (stabilized with MEHQ) | 2873-97-4 |
| #0066 | 1,2-Cyclohexanediol (cis- and trans- mixture) | 931-17-9 |
| #1102 | 3-Picolylamine | 3731-52-0 |
| #0640 | 2,3-Dimethyl-1-phenyl-5-pyrazolone (antipyrine) | 60-80-0 |
| #1439 | N,N,N',N'-Tetramethylethylenediamine | 110-18-9 |
| #1242 | Sodium Hippurate | 532-94-5 |
| #0937 | N-Ethylsuccinimide | 2314-78-5 |
| #1088 | Propionamide | 79-05-0 |
| #1047 | 1-[2-(2-Hydroxyethoxy)ethyl]piperazine | 13349-82-1 |
| #0902 | 3-Methylamino-1,2-propanediol | 40137-22-2 |
| #0927 | 3-Ethyl-3-oxetanemethanol | 3047-32-3 |
| #0665 | N,N-Dimethylethylamine | 598-56-1 |
| #0498 | 2-Aminopyrazine | 5049-61-6 |
| #1261 | Morpholine | 110-91-8 |
| #0420 | 1,2-Bis(2-aminoethoxy)ethane | 929-59-9 |
| #0270 | 1-Butyl-4-methylpyridinium Chloride | 112400-86-9 |
| #0050 | 3-Acetamidopiperidine | 5810-55-9 |

**[Table 5]**

| ID | Compound name | CAS No. |
|---|---|---|
| #0773 | N,N-Diethylformamide | 617-84-5 |
| #0604 | N, N-D imethylethylenediamine | 108-00-9 |
| #0024 | 2-(2-Aminoethoxy)ethanol | 929-06-6 |
| #1292 | Ethyl N-Methylcarbamate | 105-40-8 |
| #0912 | 2-Methylthiazole | 3581-87-1 |
| #0653 | Disodium Diphenylsulfone-4,4'-dichloro-3,3'-disulfonate | 51698-33-0 |
| #0484 | 1-(3-Aminopropyl)imidazole | 5036-48-6 |
| #0976 | Sodium Heptanoate | 10051-45-3 |
| #1338 | N,N-Dimethylacrylamide | 2680-03-7 |
| #0910 | 2,2'-Diamino-N-methyldiethylamine | 4097-88-5 |
| #1404 | Tetrahydrofurfurylamine | 4795-29-3 |
| #0900 | 3-Hydroxy-1-methylpiperidine | 3554-74-3 |
| #1265 | Methylglyoxime | 1804-15-5 |
| #1226 | 1-(2-Hydroxyethyl)-4-(3-hydroxypropyl)piperidine | 19780-85-9 |
| #0908 | Dimethyl Sulfone | 67-71-0 |
| #1508 | Dimethylacetamide | 127-19-5 |
| #0325 | 1-Amino-2-butanol | 13552-21-1 |
| #0324 | DL-2-Amino-1-butanol | 96-20-8 |
| #0903 | 3-Sulfopropyl Methacrylate Potassium Salt | 31098-21-2 |
| #1086 | Pyrazine | 290-37-9 |
| #0943 | N-Methylformamide | 123-39-7 |
| #1060 | 4-Hydroxy-2-butanone | 590-90-9 |
| #0657 | N,N-Diethylacrylamide | 2675-94-7 |
| #0835 | 1,4-Benzenedimethanol | 589-29-7 |
| #0195 | Quinaldic Acid Sodium | 16907-79-2 |
| #0367 | 2-Amino-2-methyl-1,3-propanediol | 115-69-5 |
| #1279 | 2-Methylimidazole | 693-98-1 |
| #0679 | 2,4-Diaminopyrimidine | 156-81-0 |
| #1179 | Sodium n-Octanoate | 1984-06-1 |
| #1262 | N-Methylthiourea | 598-52-7 |

**[Table 6]**

| ID | Compound name | CAS No. |
|---|---|---|
| #1516 | Ethanol | 64-17-5 |
| #1184 | Sodium 1-Naphthaleneacetate | 61-31-4 |
| #0977 | 2-Ethylimidazole | 1072-62-4 |
| #0439 | Sodium Benzenesulfinate Dihydrate | 25932-11-0 |
| #1176 | N,N,N',N",N"-Pentamethyldiethylenetriamine | 3030-47-5 |
| #0309 | Bis(2-hydroxyethyl)aminotris(hydroxymethyl) methane | 6976-37-0 |
| #0263 | 1-Butyl-4-methylpyridinium Bromide | 65350-59-6 |
| #0947 | 4-Formylmorpholine | 4394-85-8 |
| #1451 | Triethylenetetramine | 112-24-3 |
| #0461 | 3-Amino-5-methylthio-1H-1,2,4-triazole | 45534-08-5 |
| #1069 | Sodium Pyridine-3-sulfonate | 15521-77-4 |
| #0616 | Sodium 2,4-Dichlorophenoxyacetate Monohydrate | 2702-72-9 |
| #0668 | 1-(2-Dimethylaminoethyl)-4-methylpiperazine | 104-19-8 |
| #1036 | Sodium 1-Pentanesulfonate | 22767-49-3 |
| #0614 | N-N itrosodiethylamine | 55-18-5 |
| #0998 | Isonipecotamide | 39546-32-2 |
| #1603 | Hydrazine Monohydrate | 7803-57-8 |
| #1029 | Sodium 2-Naphthalenesulfonate | 532-02-5 |
| #0887 | Maltitol | 585-88-6 |
| #1031 | Sodium 1-Butanesulfonate | 2386-54-1 |
| #1349 | 4-(4-Hydroxybutyl)thiomorpholine 1,1-Dioxide | 59801-41-1 |
| #0275 | 4-tert-Butyl-1-(3-sulfopropyl)pyridinium Hydroxide Inner Salt Hydrate | 570412-84-9 |
| #0440 | Sodium Benzenesulfonate | 515-42-4 |
| #1106 | 2,6-Pyridinedimethanol | 1195-59-1 |
| #0414 | N-Butyldiethanolamine | 102-79-4 |
| #0778 | N,N-Diethylisonicotinamide | 530-40-5 |
| #1422 | Veratryl Alcohol | 93-03-8 |
| #0619 | N,N'-Diacetylethylenediamine | 871-78-3 |
| #1352 | Imidazole | 288-32-4 |
| #0975 | Sodium 4-Ethylbenzenesulfonate | 14995-38-1 |

**[Table 7]**

| ID | Compound name | CAS No. |
|---|---|---|
| #1490 | 2-Sulfobenzaldehyde Sodium | 1008-72-6 |
| #0409 | 1-Butanol | 71-36-3 |
| #1427 | Tetrahydrofuran | 109-99-9 |
| #0354 | Adipic Dihydrazide | 1071-93-8 |
| #1034 | Tetrabutylammonium Bromide | 1643-19-2 |
| #1600 | 3,3'-Iminodipropionitrile | 111-94-4 |
| #1429 | Sulfamide | 7803-58-9 |
| #0416 | Bis(2-methoxyethyl)amine | 111-95-5 |
| #1570 | Ethylene Cyanohydrin | 109-78-4 |
| #1092 | Piperazine Hexahydrate | 142-63-2 |
| #1011 | Lactamide | 2043-43-8 |
| #0788 | N,N-Dimethylbenzamide | 611-74-5 |
| #1105 | N,N,N',N",N"-Pentakis(2-hydroxypropyl)diethylenetriamine | 17121-34-5 |
| #1440 | Sodium p-Toluenesulfonate | 657-84-1 |
| #0771 | 2-Cyclohexylaminoethanesulfonic Acid | 103-47-9 |
| #1234 | 1,6-Hexanediol | 629-11-8 |
| #0419 | N-tert-Butyl-2-methoxyethylamine | ( 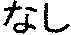 ) |
| #1268 | N-Methylacetamide | 79-16-3 |
| #0005 | Triethanolamine | 102-71-6 |
| #0351 | Acetamide | 60-35-5 |
| #1373 | N,N,N',N'-Tetrakis(2-hydroxypropyl)ethylenediamine | 102-60-3 |
| #1258 | 1-Methylpiperazine | 109-01-3 |
| #0906 | 1-Methylpyridinium Chloride | 7680-73-1 |
| #1167 | 1-(2- Pyrimidyl) pipe razine | 20980-22-7 |
| #1059 | 2-Hydroxyethyl Methyl Sulfone | 15205-66-0 |
| #1586 | Ethylene Cyanohydrin | 109-78-4 |
| #1565 | Chlorocholine Chloride | 999-81-5 |
| #0304 | Benzyltrimethylammonium Bromide | 5350-41-4 |
| #0337 | 2-(2-Aminoethylamino)ethanol | 111-41-1 |
| #1353 | N-Isopropylacrylamide | 2210-25-5 |
| #1174 | Sodium Isonicotinate | 16887-79-9 |

**[Table 8]**

| ID | Compound name | CAS No. |
|---|---|---|
| #0321 | Amylamine | 110-58-7 |
| #0486 | 1-Amino-2-propanol | 78-96-6 |
| #0388 | (+)-3-Bromocamphor-8-sulfonic Acid Ammonium | 14575-84-9 |
| #0322 | 3-Amino-1,2-propanediol | 616-30-8 |
| #1260 | N-Methyldiethanolamine | 105-59-9 |
| #1000 | Isopropanolamine Phosphate | 67952-32-3 |
| #0324 | DL-2-Amino-1-butanol | 96-20-8 |
| #1047 | 1-[2-(2-H ydroxyethoxy) ethyl]piperazine | 13349-82-1 |
| #0665 | N,N-Dimethylethylamine | 598-56-1 |
| #0902 | 3-Methylamino-1,2-propanediol | 40137-22-2 |
| #1226 | 1-(2-Hydroxyethyl)-4-(3-hydroxypropyl)piperidine | 19780-85-9 |
| #0604 | N,N-Dimethylethylenediamine | 108-00-9 |
| #0420 | 1,2-Bis(2-aminoethoxy)ethane | 929-59-9 |
| #0024 | 2-(2-Aminoethoxy)ethanol | 929-06-6 |
| #1258 | 1-Methylpiperazine | 109-01-3 |
| #1439 | N,N,N',N'-Tetramethylethylenediamine | 110-18-9 |
| #0910 | 2,2'-Diamino-N-methyldiethylamine | 4097-88-5 |
| #1404 | T etrahydrofurf urylamine | 4795-29-3 |
| #0325 | 1-Amino-2-butanol | 13552-21-1 |
| #0050 | 3-Acetamidopiperidine | 5810-55-9 |
| #0146 | 2-[2-(Dimethylamino)ethoxy]ethanol | 1704-62-7 |
| #1091 | 2-Piperidone | 675-20-7 |
| #1376 | Thiomorpholine | 123-90-0 |
| #0900 | 3-Hydroxy-1-methylpiperidine | 3554-74-3 |
| #0484 | 1-(3-Aminopropyl)imidazole | 5036-48-6 |
| #0640 | 2,3-Dimethyl-1-phenyl-5-pyrazolone (antipyrine) | 60-80-0 |
| #0428 | 1,3-Bis[tris(hydroxymethyl)methylamino]propane | 64431-96-5 |
| #0444 | o-Sulfobenzimide Sodium Dihydrate | 128-44-9 |
| #0493 | 4-(2-Aminoethyl) morpholine | 2038-03-1 |

### Example 16: 3D Immunostaining with Representative Combinations of Additives

A cerebral hemisphere defatted in the same manner as in Example 1 was soaked in Immunostaining Buffer E, and staining was carried out at 32°C for 3 days to 4 days. The immunostaining Buffer E contains: an anti-synaptophysin antibody (#D073-3; 20 µg/mL), mouse monoclonal anti-Gad67 IgG₂ₐ (manufactured by Merck Millipore; #MAB5406; 10 µg/mL), mouse monoclonal anti-Dat IgG₁ (manufactured by abcam; #ab128848; 10 µg/mL), or a mouse monoclonal anti-Th antibody (manufactured by abeam; #13786; 10 µg/mL) as a primary antibody; Fab-anti-mouse or rabbit IgG against each primary antibody, labeled with Alexa 594 (manufactured by Jackson ImmunoResearch laboratories; Fab-anti-mouse IgG₁-A594 (#115-587-185), Fab-anti-mouse IgG₂ₐ-A594 (#115-587-186), or Fab-anti-rabbit IgG-A594 (#111-587-008), added such that the weight ratio to each primary antibody was about 1 :0.75); and predetermined concentrations of additives (two or three selected from #0854, #1086, and #0609 described in FIG. 1A and #0146 described in FIG. 1B). Frozen sections prepared after the staining were observed in the same manner as in Example 1.

### (Results)

According to FIG. 20A, B, C, and D, which show images of frozen sections of the samples stained with the antibodies against synaptophysin, Gad67, Dat, or Th, respectively, the staining performance inside each sample increased by combination of the additives. The penetration-promoting effect for the antibodies by the additives was thus demonstrated. In particular, as shown in the staining example with the anti-Th antibody, use of the additive #0146 resulted in production of an even stronger penetration-promoting effect.

### Example 17: 3D Nuclear Staining with Representative Combinations of Additives

A half brain defatted in the same manner as in Example 1 was soaked in 250 µL of Immunostaining Buffer F, and staining was carried out at 37°C for 2 days or 3 days. The immunostaining Buffer F contains RedDot2 (1:50) or SYTOX-G (1:500), and predetermined concentrations of additives (two selected from #0854, #1086, and #0609 shown in FIG. 1A). Thereafter, from each sample, a frozen section was prepared in the same manner as in Example 2. The obtained frozen section was observed under a fluorescence microscope in the same manner as in Example 1.

### (Results)

According to FIG. 21A and B, which show nuclear staining images of frozen sections of the samples stained with RedDot2 or SYTOX-G, respectively, the staining performance inside each sample increased by combination of the additives. The increase in the staining performance was especially remarkable in the cerebellar region. The present Example demonstrated the penetration-promoting effect of the additives on the dyes.

### Example 18: Simultaneous 3D Nuclear Staining and 3D Immunostaining with Representative Combinations of Additives

### (Sample Preparation)

The whole brain of an 8-week-old C57BL/6 mouse fixed in the same manner as in Example 1 was soaked in CUBIC-L at 37°C for 3 days, to obtain a defatted sample. The sample was washed with PBS, and stored in PBS containing 0.05% NaN₃ at 4°C.

### (3D Nuclear Staining and 3D Immunostaining)

The sample was soaked in 500 µL of Immunostaining Buffer F supplemented with SYTOX-G (1:500), 10 µg/mL mouse anti-NeuN antibody, Fab-anti-mouse IgG₁ labeled with Alexa 594 (#115-587-185; added such that the weight ratio to the mouse anti-NeuN antibody was about 1:0.75), 2.5% by weight nicotinic acid hydrazide (#0854), and 5% by weight pyrazine (#1086). The sample was stained at 32°C for 6 days, and then at 4°C for 1 day. The stained sample was cleared with CUBIC-R (RI adjustment) in the same manner as in Example 9, and embedded in a gel prepared with 2% (w/v) agarose dissolved in CUBIC-R, followed by 3D observation using LSFM. The brain image obtained using LSFM was tiled using Fiji/ImageJ in the same manner as in Example 9. The z-stack data obtained were reconstructed using Imaris software, to visualize the data as a pseudocolor image. In the present Example, the enzyme treatment in Example 9 was not carried out.

### (Results)

FIG. 22A is an image showing the signal of NeuN. FIG. 22B is an image showing the signal of SYTOX-G. FIG. 22C is an image prepared by merging the image showing the signal of NeuN and the image showing the signal of SYTOX-G. The image corresponds to a stack in the central portion of the sample extracted from the z-stack data obtained by LSFM, which stack allows evaluation of the penetrability of the dyes. As shown in FIG. 22A to C, uniformly stained images could be acquired at a cellular-level resolution. The results demonstrated that the use of the additives enables nuclear staining and immunostaining of a mouse whole brain.

### Example 19: 3D Immunostaining Using Phase Separation

A sample obtained from a cerebellar hemisphere defatted in the same manner as in Example 1 was soaked, with or without phase separation, in Immunostaining Buffer E, or in PBS mixed with 0.1% Triton X-100 (hereinafter referred to as "PBST"). Staining was carried out at 32°C for 2 days. Frozen sections prepared after the staining were observed using a fluorescence microscope.

Each of the immunostaining Buffer E and the PBST contains mouse monoclonal anti-NeuN IgG (10 µg/mL or 100 µg/mL) and Fab-anti-mouse IgG₁ labeled with Alexa 594 (#115-587-185; added such that the weight ratio to the mouse anti-NeuN antibody was about 1:0.75). Under the conditions without phase separation, the amount of the Immunostaining Buffer E or the PBST used was set to 150 µL. On the other hand, under the conditions with phase separation, the amount of the Immunostaining Buffer E or the PBST used was set to 15 µL, and phase separation of the sample and the Immunostaining Buffer E or the PBST was allowed to occur in silicone oil KF-96 (viscosity, 50).

### (Results)

An image of the frozen section of each sample is shown in FIG. 23. Under both of the conditions with and without phase separation, antibody penetration increased depending on the initial concentration and the total amount of the antibody, resulting in enhancement of the signal. For the cases where the total amount of the antibody per mouse whole brain was 5 µg, a comparison was made between the conditions where the antibody was used at a concentration of 10 µg/mL without allowing phase separation and the conditions where the antibody was used at a concentration of 100 µg/mL allowing phase separation. The comparison demonstrated that, by increasing the initial concentration by phase separation, the signal can be enhanced due to an increased antibody penetration. By the use of the phase separation, enhancement of the signal was possible even with the same total amount of antibody, that is, at the same cost, due to the increased antibody penetration.

### Example 20: Simultaneous 3D Nuclear Staining and 3D Immunostaining Using Additives and Phase Separation

### (Sample Preparation)

A mouse whole-brain sample defatted in the same manner as in Example 1 was washed with PBS, and stored in PBS containing 0.05% NaN₃ at 4°C until use.

### (3D Nuclear Staining and 3D Immunostaining)

The sample was soaked in 50 µL of Staining Buffer F supplemented with SYTOX-G (1:50), a mouse monoclonal anti-GFAP antibody (manufactured by Medical & Biological Laboratories Co., Ltd.; #D097-3; 100 µg/mL) or a mouse monoclonal anti-Dat antibody (manufactured by abcam; #ab128848; 100 µg/mL), Fab-anti-mouse IgG₁ labeled with Alexa 594 (in an amount corresponding to a weight ratio of about 1:0.75 to the primary antibody), 2.5% by weight nicotinic acid hydrazide (#0854), and 5% by weight pyrazine (#1086). Their phase separation was allowed to occur in mineral oil, and staining was carried out at 32°C for 5 days, and then at 4°C for 1 day. The stained sample was cleared with CUBIC-R (RI adjustment) in the same manner as in Example 9, and embedded in a gel prepared with 2% (w/v) agarose dissolved in CUBIC-R, followed by 3D observation using LSFM. The brain image obtained using LSFM was tiled using Fiji/ImageJ in the same manner as in Example 9. The z-stack data obtained were reconstructed using Imaris software, to visualize the data as a pseudocolor image. In the present Example, the enzyme treatment in Example 9 was not carried out.

### (Results)

FIG. 24A shows a three-dimensional reconstructed image showing the signals of GFAP and SYTOX-G. FIG. 24B shows an image of the signal of GFAP for demonstration of the internal staining performance, which image was prepared by extracting the z-stack in the central portion of the sample followed by superimposition at the maximum brightness value (MAX). FIG. 24C shows a three-dimensional reconstructed image showing the signals of Dat and SYTOX-G. FIG. 24D shows an image of the signal of Dat, which image was prepared by extracting the z-stack in the central portion of the sample followed by superimposition at the maximum brightness value (MAX). The results demonstrated that, by using both the additives and the phase separation for concentration of the antibody and the dye, uniform nuclear staining and immunostaining can be completed within 1 week.

### Industrial Applicability

The present disclosure is suitable for staining of biological tissues.

## Claims

1. A biological tissue-staining reagent comprising:
a nonionic surfactant at a concentration higher than 1%; and
a salt at not less than 200 mM.

2. The biological tissue-staining reagent according to claim 1, further comprising:
a neutral buffer.

3. The biological tissue-staining reagent according to claim 1 or 2, further comprising:
a blocking reagent.

4. The biological tissue-staining reagent according to any one of claims 1 to 3, further comprising:
at least one additive selected from compounds of aromatic amines, aliphatic amides, nicotinamides, sulfamides, sulfonic acid salts, amino alcohols, alcohols, sulfinic acids, thioureas, and carboxylic acids except urea and urea derivatives, wherein
the compounds are represented by the following General Formula (1): 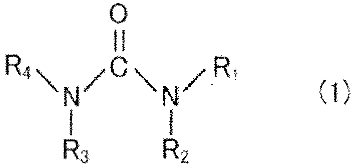 (where in General Formula (1), R₁, R₂, R₃, and R₄ are each independently a hydrogen atom, a halogen atom, or a hydrocarbon group, wherein, in a case where a plurality of carbon atoms constitutes the hydrocarbon group, part of the carbon atoms are optionally substituted by a heteroatom such as a nitrogen atom, an oxygen atom, or a sulfur atom, and wherein the hydrocarbon group includes chain hydrocarbon groups and cyclic hydrocarbon groups).

5. The biological tissue-staining reagent according to any one of claims 1 to 4, further comprising:
a dye.

6. The biological tissue-staining reagent according to any one of claims 1 to 5, further comprising:
an immunostaining antibody.

7. A biological tissue-staining kit comprising:
the biological tissue-staining reagent according to any one of claims 1 to 4; and a dye.

8. A biological tissue-staining kit comprising:
the biological tissue-staining reagent according to any one of claims 1 to 4; and
an immunostaining antibody.

9. The biological tissue-staining kit according to claim 8, further comprising:
a slightly acidic buffer.

10. The biological tissue-staining kit according to claim 8 or 9, further comprising:
a washing buffer containing a nonionic surfactant at a concentration higher than 1%, a salt at not less than 200 mM, and a neutral buffer.

11. The biological tissue-staining kit according to any one of claims 7 to 10, further comprising:
a phase separation-inducing reagent for phase separation from water.

12. A biological tissue-staining method using the biological tissue-staining reagent according to claim 6, the method comprising:
exposing a defatted biological tissue to a slightly acidic buffer containing the immunostaining antibody; and
exposing the biological tissue to the biological tissue-staining reagent.

13. A biological tissue-staining method comprising:
exposing a biological tissue to a biological tissue-staining reagent according to claim 4, the reagent comprising a dye and an immunostaining antibody.

14. A biological tissue-staining method comprising:
mixing the biological tissue-staining reagent according to any one of claims 1 to 4, the reagent comprising at least one of a dye or an immunostaining antibody, a biological tissue, and a phase separation-inducing reagent.

## Patentansprüche

1. Reagenz zum Färben von biologischem Gewebe, umfassend:
ein nichtionisches Tensid in einer Konzentration von mehr als 1 %; und
ein Salz in einer Konzentration von nicht weniger als 200 mM.

2. Reagenz zum Färben von biologischem Gewebe gemäß Anspruch 1, ferner umfassend:
einen neutralen Puffer.

3. Reagenz zum Färben von biologischem Gewebe gemäß Anspruch 1 oder 2, ferner umfassend:
ein Blockierungsreagenz.

4. Reagenz zum Färben von biologischem Gewebe gemäß irgendeinem der Ansprüche 1 bis 3, ferner umfassend:
mindestens ein Additiv, ausgewählt aus Verbindungen von aromatischen Aminen, aliphatischen Amiden, Nicotinamiden, Sulfamiden, Sulfonsäuresalzen, Aminoalkoholen, Alkoholen, Sulfinsäuren, Thioharnstoffen und Carbonsäuren, ausgenommen Harnstoff und Harnstoffderivate, wobei
die Verbindungen durch die folgende allgemeine Formel (1) dargestellt werden: 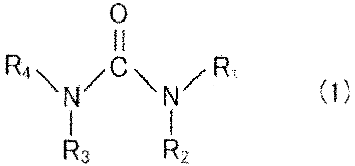 (wobei in der allgemeinen Formel (1) R₁, R₂, R₃ und R₄ jeweils unabhängig voneinander ein Wasserstoffatom, ein Halogenatom oder eine Kohlenwasserstoffgruppe sind, wobei in einem Fall, in dem eine Vielzahl von Kohlenstoffatomen die Kohlenwasserstoffgruppe bildet, ein Teil der Kohlenstoffatome optional durch ein Heteroatom, wie zum Beispiel ein Stickstoffatom, ein Sauerstoffatom oder ein Schwefelatom, substituiert ist, und wobei die Kohlenwasserstoffgruppe kettenförmige Kohlenwasserstoffgruppen und zyklische Kohlenwasserstoffgruppen umfasst).

5. Reagenz zum Färben von biologischem Gewebe gemäß irgendeinem der Ansprüche 1 bis 4, ferner umfassend:
einen Farbstoff.

6. Reagenz zum Färben von biologischem Gewebe gemäß irgendeinem der Ansprüche 1 bis 5, ferner umfassend:
einen Antikörper für die Immunfärbung.

7. Kit zum Färben von biologischem Gewebe, umfassend:
das Reagenz zum Färben von biologischem Gewebe gemäß irgendeinem der Ansprüche 1 bis 4; und
einen Farbstoff.

8. Kit zum Färben von biologischem Gewebe, umfassend:
das Reagenz zum Färben von biologischem Gewebe gemäß irgendeinem der Ansprüche 1 bis 4; und
einen Antikörper für die Immunfärbung.

9. Kit zum Färben von biologischem Gewebe gemäß Anspruch 8, ferner umfassend:
einen leicht sauren Puffer.

10. Kit zum Färben von biologischem Gewebe gemäß Anspruch 8 oder 9, ferner umfassend:
einen Waschpuffer, der ein nichtionisches Tensid in einer Konzentration von mehr als 1 %, ein Salz in einer Konzentration von nicht weniger als 200 mM und einen neutralen Puffer enthält.

11. Kit zum Färben von biologischem Gewebe gemäß irgendeinem der Ansprüche 7 bis 10, ferner umfassend:
ein Phasentrennung-induzierendes Reagenz für die Phasentrennung von Wasser.

12. Verfahren zum Färben von biologischem Gewebe unter Verwendung des Reagenz zum Färben von biologischem Gewebe gemäß Anspruch 6, wobei das Verfahren umfasst:
Aussetzen eines entfetteten biologischen Gewebes einem leicht sauren Puffer, der den Antikörper für die Immunfärbung enthält; und
Aussetzen des biologischen Gewebes dem Reagenz zum Färben von biologischem Gewebe.

13. Verfahren zum Färben von biologischem Gewebe, umfassend:
Aussetzen eines biologischen Gewebes einem Reagenz zum Färben von biologischem Gewebe gemäß Anspruch 4, wobei das Reagenz einen Farbstoff und einen Antikörper für die Immunfärbung umfasst.

14. Verfahren zum Färben von biologischem Gewebe, umfassend:
Mischen des Reagenz zum Färben von biologischem Gewebe gemäß irgendeinem der Ansprüche 1 bis 4, wobei das Reagenz einen Farbstoff und/oder einen Antikörper für die Immunfärbung umfasst, eines biologischen Gewebes und eines Phasentrennung-induzierenden Reagenz.

## Revendications

1. Réactif de coloration de tissu biologique comprenant :
un agent tensioactif non ionique à une concentration supérieure à 1% ; et
un sel à pas moins de 200 mM.

2. Réactif de coloration de tissu biologique selon la revendication 1, comprenant en outre :
un tampon neutre.

3. Réactif de coloration de tissu biologique selon la revendication 1 ou 2, comprenant en outre :
un réactif de blocage.

4. Réactif de coloration de tissu biologique selon l'une quelconque des revendications 1 à 3, comprenant en outre :
au moins un additif choisi parmi les composés d'amines aromatiques, d'amides aliphatiques, de nicotinamides, de sulfamides, de sels d'acide sulfonique, d'aminoalcools, d'alcools, d'acides sulfiniques, de thiourées et d'acides carboxyliques à l'exception de l'urée et des dérivées d'urée, dans lequel
les composés sont représentés par la formule générale (1) suivante : 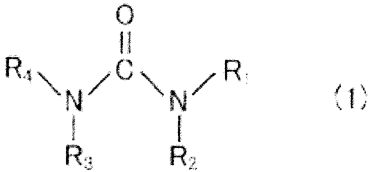 (où dans la formule générale (1), R₁, R₂, R₃ et R₄ sont chacun indépendamment un atome d'hydrogène, un atome d'halogène ou un groupe d'hydrocarbures, dans lequel, dans le cas où une pluralité d'atomes de carbone constitue le groupe d'hydrocarbures, une partie des atomes de carbone est facultativement substituée par un hétéroatome tel qu'un atome d'azote, un atome d'oxygène ou un atome de soufre, et dans lequel le groupe d'hydrocarbures inclut les groupes d'hydrocarbures à chaîne et les groupes d'hydrocarbures cycliques).

5. Réactif de coloration de tissu biologique selon l'une quelconque des revendications 1 à 4, comprenant en outre :
un colorant.

6. Réactif de coloration de tissu biologique selon l'une quelconque des revendications 1 à 5, comprenant en outre :
un anticorps d'immunocoloration.

7. Kit de coloration de tissu biologique comprenant :
le réactif de coloration de tissu biologique selon l'une quelconque des revendications 1 à 4 ; et
un colorant.

8. Kit de coloration de tissu biologique comprenant :
le réactif de coloration de tissu biologique selon l'une quelconque des revendications 1 à 4 ; et
un anticorps d'immunocoloration.

9. Kit de coloration de tissu biologique selon la revendication 8, comprenant en outre :
un tampon légèrement acide.

10. Kit de coloration de tissu biologique selon la revendication 8 ou 9, comprenant en outre :
un tampon de lavage contenant un agent tensioactif non ionique à une concentration supérieure à 1%, un sel à pas moins de 200 mM et un tampon neutre.

11. Kit de coloration de tissu biologique selon l'une quelconque des revendications 7 à 10, comprenant en outre :
un réactif d'induction de séparation de phase pour la séparation de phase de l'eau.

12. Méthode de coloration de tissu biologique utilisant le réactif de coloration de tissu biologique selon la revendication 6, la méthode comprenant :
l'exposition d'un tissu biologique dégraissé à un tampon légèrement acide contenant l'anticorps d'immunocoloration ; et
l'exposition du tissu biologique au réactif de coloration de tissu biologique.

13. Méthode de coloration de tissu biologique comprenant :
l'exposition d'un tissu biologique à un réactif de coloration de tissu biologique selon la revendication 4, le réactif comprenant un colorant et un anticorps d'immunocoloration.

14. Méthode de coloration de tissu biologique comprenant :
le mélange du réactif de coloration de tissu biologique selon l'une quelconque des revendications 1 à 4, le réactif comprenant au moins l'un d'un colorant ou d'un anticorps d'immunocoloration, un tissu biologique et un réactif d'induction de séparation de phase.
